(19) Europäisches Patentamt
European Patent Office
Office européen des brevets

(11) EP 4 310 090 A1

(12) **EUROPEAN PATENT APPLICATION**
published in accordance with Art. 153(4) EPC

(43) Date of publication:
**24.01.2024 Bulletin 2024/04**

(21) Application number: **22771279.1**

(22) Date of filing: **10.03.2022**

(51) International Patent Classification (IPC):
***C07D 491/044*** *(2006.01)* ***A01P 13/00*** *(2006.01)*
***C07D 519/00*** *(2006.01)* ***A01N 43/58*** *(2006.01)*
***A01N 43/653*** *(2006.01)* ***A01N 43/707*** *(2006.01)*

(52) Cooperative Patent Classification (CPC):
**A01N 43/58; A01N 43/653; A01N 43/707; A01P 13/00; C07D 491/044; C07D 519/00**

(86) International application number:
**PCT/JP2022/010651**

(87) International publication number:
**WO 2022/196528 (22.09.2022 Gazette 2022/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.03.2021 JP 2021046669**

(71) Applicant: **Nippon Soda Co., Ltd.**
**Tokyo 100-7010 (JP)**

(72) Inventors:
- **MIHARA Ken**
**Odawara-shi, Kanagawa 250-0280 (JP)**
- **IKEDA Yoji**
**Odawara-shi, Kanagawa 250-0280 (JP)**
- **HORIKOSHI Humberto Mitio**
**Odawara-shi, Kanagawa 250-0280 (JP)**
- **KATO Kazushige**
**Odawara-shi, Kanagawa 250-0280 (JP)**
- **INAGAKI Jun**
**Odawara-shi, Kanagawa 250-0280 (JP)**
- **HORI Masahiro**
**Odawara-shi, Kanagawa 250-0280 (JP)**
- **SUGIYAMA Hirotaka**
**Odawara-shi, Kanagawa 250-0280 (JP)**

(74) Representative: **Mewburn Ellis LLP**
**Aurora Building**
**Counterslip**
**Bristol BS1 6BX (GB)**

(54) **7-OXA-3,4-DIAZABICYCLO[4.1.0]HEPTA-4-ENE-2-ONE COMPOUND AND HERBICIDE**

(57) A compound represented by a formula (I) or a salt thereof; and a herbicide containing at least one selected from them as an active ingredient:

[Chemical Formula 1]

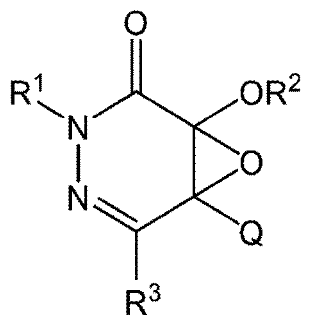

(I)

wherein $R^1$ represents a substituted or unsubstituted $C_{1-6}$ alkyl group or the like, $R^2$ represents a substituted or unsubstituted $C_{1-6}$ alkyl group or the like, $R^3$ represents a hydrogen atom, a substituted or unsubstituted $C_{1-6}$ alkyl group or the like, and Q represents a substituted or unsubstituted 5- to 10-membered heterocyclyl group.

EP 4 310 090 A1

## Description

BACKGROUND OF THE INVENTION

Field of the Invention

The present invention relates to a 7-oxa-3,4-diazabicyclo[4.1.0]hept-4-en-2-one compound and a herbicide. More specifically, the present invention relates to a 7-oxa-3,4-diazabicyclo[4.1.0]hept-4-en-2-one compound useful as an active ingredient of a herbicide, which has a reliable weed control effect even at a low dose, causes less phytotoxicity to crops (useful plants), and is highly safe for the environment; and a herbicide.

Priority is claimed on Japanese Patent Application No. 2021-046669, filed March 19, 2021, the content of which is incorporated herein by reference.

Description of the Related Art

In the cultivation of agricultural and horticultural crops, herbicides may be used for controlling weeds. Various compounds have been proposed so far as active ingredients of herbicides.

For example, Patent Document 1 discloses a compound represented by a formula (A), and the like.

[Chemical Formula 1]

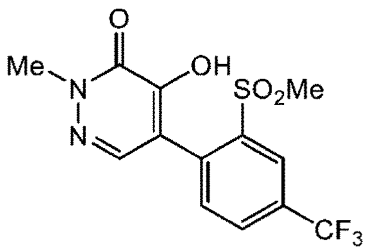

(A)

PRIOR ART DOCUMENTS

[Patent Document]

[Patent Document 1] WO2013 / 050421A1

SUMMARY OF THE INVENTION

Problems to be Solved by the Invention

Herbicides are required not only to have an excellent weed control effect, but also to have low phytotoxicity to crops, to be less likely to remain in the environment, and not to pollute the environment.

An object of the present invention is to provide a 7-oxa-3,4-diazabicyclo[4.1.0]hept-4-en-2-one compound useful as an active ingredient of a herbicide, which has a reliable weed control effect even at a low dose, causes less phytotoxicity to crops, and is highly safe for the environment; and a herbicide.

Means for Solving the Problem

As a result of intensive studies in order to achieve the above object, the present invention including the following embodiments has been completed.

[1] A compound represented by a formula (I) or a salt thereof:

[Chemical Formula 2]

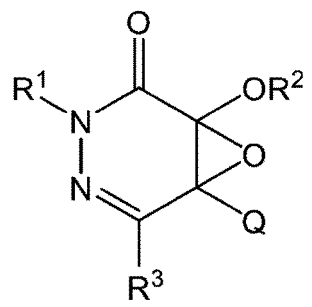

(I)

In the formula (I),

$R^1$ represents a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{3-6}$ cycloalkyl group, or a 5- to 6-membered cyclic ether group,
$R^2$ represents a substituted or unsubstituted $C_{1-6}$ alkyl group, a substituted or unsubstituted $C_{2-6}$ alkenyl group, or a substituted or unsubstituted $C_{2-6}$ alkynyl group,
$R^3$ represents a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{3-6}$ cycloalkyl group, or a substituted or unsubstituted phenyl group, and
Q represents a substituted or unsubstituted 5- to 10-membered heterocyclyl group.

[2] A herbicide containing at least one selected from the group consisting of the compound according to [1] and a salt thereof as an active ingredient.
[3] A method for controlling a weed, the method including a step of applying the compound according to [1] or a salt thereof, or the herbicide according to [2] to a useful plant, a weed in the aforementioned useful plant, and/or a place where the useful plant grows or is growing.

Effects of the Invention

Since the 7-oxa-3,4-diazabicyclo[4.1.0]hept-4-en-2-one compound of the present invention has a reliable weed control effect even at a low dose, causes less phytotoxicity to useful crops, and is highly safe for the environment, it is useful as an active ingredient of a herbicide. The herbicide of the present invention can be safely used for weed control in the cultivation of useful agricultural and horticultural crops.

DETAILED DESCRIPTION OF THE INVENTION

The 7-oxa-3,4-diazabicyclo[4.1.0]hept-4-en-2-one compound of the present invention (hereinafter, may be referred to as "the compound of the present invention" for simplicity) is a compound represented by the formula (I) (sometimes referred to as compound (I)) or a salt of the compound (I). Examples of the salt of the compound (I) include salts of alkali metals such as lithium, sodium and potassium; salts of alkaline earth metals such as calcium and magnesium; salts of transition metals such as iron and copper; ammonium salts; and salts of organic bases such as triethylamine, tributylamine, pyridine and hydrazine. The compound (I) also includes hydrates, various types of solvates, polymorphs of crystals, and the like. The compound (I) may have stereoisomers based on asymmetric carbons, double bonds and the like, and tautomers. All such isomers and mixtures thereof are within the technical scope of the present invention. The structure of the compound (I) or the salt of the compound (I) can be determined by NMR spectra, IR spectra, MS spectra and the like.
The term "unsubstituted" used in the present specification means that it is composed only of a group which becomes a mother nucleus. When it is described only by the name of the group which becomes the mother nucleus without being described as "substituted", it means "unsubstituted" unless otherwise stated.
On the other hand, the term "substituted" means that any hydrogen atom of the group which becomes a mother nucleus is substituted with a group (substituent) having the same or different structure as that of the mother nucleus. Therefore, a "substituent" is another group bonded to a group which becomes a mother nucleus. The number of substituents may be one, or two or more. The two or more substituents may be the same or different.

The terms "$C_{1-6}$" and the like mean that the number of carbon atoms in the group which becomes a mother nucleus is 1 to 6, and so on. The number of carbon atoms does not include the number of carbon atoms present in the substituent. For example, a butyl group having an ethoxy group as a substituent is classified as a C2 alkoxy C4 alkyl group.

A "substituent" is not particularly limited as long as it is chemically acceptable and has the effects of the present invention. Hereinafter, groups which can be a "substituent" are exemplified.

A $C_{1-6}$ alkyl group such as a methyl group, an ethyl group, an n-propyl group, an i-propyl group, an n-butyl group, an s-butyl group, an i-butyl group, a t-butyl group, an n-pentyl group, and an n-hexyl group;
a $C_{2-6}$ alkenyl group such as a vinyl group, a 1-propenyl group, a 2-propenyl group (allyl group), a 1-butenyl group, a 2-butenyl group, a 3-butenyl group, a 1-methyl-2-propenyl group, and a 2-methyl-2-propenyl group;
a $C_{2-6}$ alkynyl group such as an ethynyl group, a 1-propynyl group, a 2-propynyl group, a 1-butynyl group, a 2-butynyl group, a 3-butynyl group, and a 1-methyl-2-propynyl group;
a $C_{3-6}$ cycloalkyl group such as a cyclopropyl group, a cyclobutyl group, a cyclopentyl group and a cyclohexyl group;
a phenyl group and a naphthyl group;
a phenyl $C_{1-6}$ alkyl group such as a benzyl group and a phenethyl group;
a 3- to 6-membered heterocyclyl group;
a 3- to 6-membered heterocyclyl $C_{1-6}$ alkyl group;
a hydroxyl group;
a $C_{1-6}$ alkoxy group such as a methoxy group, an ethoxy group, an n-propoxy group, an i-propoxy group, an n-butoxy group, an s-butoxy group, an i-butoxy group, and a t-butoxy group;
a $C_{2-6}$ alkenyloxy group such as a vinyloxy group, an allyloxy group, a propenyloxy group, and a butenyloxy group;
a $C_{2-6}$ alkynyloxy group such as an ethynyloxy group and a propargyloxy group;
a phenoxy group and a naphthoxy group;
a benzyloxy group and a phenethyloxy group;
a 5- to 6-membered heteroaryloxy group such as a thiazolyloxy group and a pyridyloxy group;
a 5- to 6-membered heteroaryl $C_{1-6}$ alkyloxy group such as a thiazolylmethyloxy group and a pyridylmethyloxy group;
a formyl group;
a $C_{1-6}$ alkylcarbonyl group such as an acetyl group and a propionyl group;
a formyloxy group;
a $C_{1-6}$ alkylcarbonyloxy group such as an acetyloxy group and a propionyloxy group;
a benzoyl group;
a $C_{1-6}$ alkoxycarbonyl group such as a methoxycarbonyl group, an ethoxycarbonyl group, an n-propoxycarbonyl group, an i-propoxycarbonyl group, an n-butoxycarbonyl group and a t-butoxycarbonyl group;
a $C_{1-6}$ alkoxycarbonyloxy group such as a methoxycarbonyloxy group, an ethoxycarbonyloxy group, an n-propoxycarbonyloxy group, an i-propoxycarbonyloxy group, an n-butoxycarbonyloxy group and a t-butoxycarbonyloxy group;
a carboxyl group;
a halogeno group such as a fluoro group, a chloro group, a bromo group, and an iodo group;
a $C_{1-6}$ haloalkyl group such as a chloromethyl group, a chloroethyl group, a difluoromethyl group, a trifluoromethyl group, a 2,2,2-trifluoroethyl group, a 1,2-dichloro-n-propyl group and a 1-fluoro-n-butyl group;
a $C_{2-6}$ haloalkenyl group such as a 2-chloro-1-propenyl group and a 2-fluoro-1-butenyl group;
a $C_{2-6}$ haloalkynyl group such as a 4,4-dichloro-1-butynyl group, a 4-fluoro-1-pentynyl group, and a 5-bromo-2-pentynyl group;
a $C_{1-6}$ haloalkoxy group such as a difluoromethoxy group, a trifluoromethoxy group, a 2,2,2-trifluoroethoxy group and a 2,3-dichlorobutoxy group;
a $C_{2-6}$ haloalkenyloxy group such as a 2-chloropropenyloxy group and a 3-bromobutenyloxy group;
a $C_{1-6}$ haloalkylcarbonyl group such as a chloroacetyl group, a trifluoroacetyl group and a trichloroacetyl group;
an amino group;
a $C_{1-6}$ alkyl-substituted amino group such as a methylamino group, a dimethylamino group and a diethylamino group;
an anilino group and a naphthylamino group;
a phenyl $C_{1-6}$ alkylamino group such as a benzylamino group and a phenethylamino group;
a formylamino group;
a $C_{1-6}$ alkylcarbonylamino group such as an acetylamino group, a propanoylamino group, a butyrylamino group and an i-propylcarbonylamino group;
a $C_{1-6}$ alkoxycarbonylamino group such as a methoxycarbonylamino group, an ethoxycarbonylamino group, an n-propoxycarbonylamino group and an i-propoxycarbonylamino group;
an unsubstituted or substituted aminocarbonyl group such as an aminocarbonyl group, a dimethylaminocarbonyl group, a phenylaminocarbonyl group and an N-phenyl-N-methylaminocarbonyl group;

an imino $C_{1-6}$ alkyl group such as an iminomethyl group, a (1-imino)ethyl group and a (1-imino)-n-propyl group;
a substituted or unsubstituted N-hydroxyimino $C_{1-6}$ alkyl group such as an N-hydroxy-iminomethyl group, a (1-(N-hydroxy)-imino)ethyl group, a (1-(N-hydroxy)-imino)propyl group, an N-methoxy-iminomethyl group, and a (1-(N-methoxy)-imino)ethyl group;
an aminocarbonyloxy group;
a $C_{1-6}$ alkyl-substituted aminocarbonyloxy group such as an ethylaminocarbonyloxy group and a dimethylaminocarbonyloxy group;
a mercapto group;
a $C_{1-6}$ alkylthio group such as a methylthio group, an ethylthio group, an n-propylthio group, an i-propylthio group, an n-butylthio group, an i-butylthio group, an s-butylthio group and a t-butylthio group;
a $C_{1-6}$ haloalkylthio group such as a trifluoromethylthio group and a 2,2,2-trifluoroethylthio group;
a phenylthio group;
a 5- to 6-membered heteroarylthio group such as a thiazolylthio group and a pyridylthio group;
a $C_{1-6}$ alkylsulfinyl group such as a methylsulfinyl group, an ethylsulfinyl group and a t-butylsulfinyl group;
a $C_{1-6}$ haloalkylsulfinyl group such as a trifluoromethylsulfinyl group and a 2,2,2-trifluoroethylsulfinyl group;
a phenylsulfinyl group;
a 5- to 6-membered heteroarylsulfinyl group such as a thiazolylsulfinyl group and a pyridylsulfinyl group;
a $C_{1-6}$ alkylsulfonyl group such as a methylsulfonyl group, an ethylsulfonyl group and a t-butylsulfonyl group;
a $C_{1-6}$ haloalkylsulfonyl group such as a trifluoromethylsulfonyl group and a 2,2,2-trifluoroethylsulfonyl group;
a phenylsulfonyl group;
a 5- to 6-membered heteroarylsulfonyl group such as a thiazolylsulfonyl group and a pyridylsulfonyl group;
a $C_{1-6}$ alkylsulfonyloxy group such as a methylsulfonyloxy group, an ethylsulfonyloxy group and a t-butylsulfonyloxy group;
a $C_{1-6}$ haloalkylsulfonyloxy group such as a trifluoromethylsulfonyloxy group and a 2,2,2-trifluoroethylsulfonyloxy group;
a tri $C_{1-6}$ alkyl-substituted silyl group such as a trimethylsilyl group, a triethylsilyl group and a t-butyldimethylsilyl group;
a triphenylsilyl group;
a pentafluorosulfanyl group;
a cyano group; and a nitro group.

Further, in these "substituents", any hydrogen atom in the substituent may be substituted with a group having a different structure. Examples of the "substituent" in this case include a $C_{1-6}$ alkyl group, a $C_{1-6}$ haloalkyl group, a $C_{1-6}$ alkoxy group, a $C_{1-6}$ haloalkoxy group, a halogeno group, a cyano group and a nitro group.

Further, the above-mentioned "3- to 6-membered heterocyclyl group" includes 1 to 4 hetero atoms selected from the group consisting of a nitrogen atom, an oxygen atom and a sulfur atom as constituent atoms of the ring. When there are two or more hetero atoms, they may be the same or different. Examples of the 3- to 6-membered heterocyclyl group include a 3- to 6-membered saturated heterocyclyl group, a 5- to 6-membered partially unsaturated heterocyclyl group, and a 5- to 6-membered heteroaryl group.

Examples of the 3- to 6-membered saturated heterocyclyl group include a 3-membered saturated heterocyclyl group such as an aziridinyl group and an oxiranyl group; a 4-membered saturated heterocyclyl group such as an azetidinyl group and an oxetanyl group; a 5-membered saturated heterocyclyl group such as a pyrrolidinyl group, a tetrahydrofuranyl group, a thiazolidinyl group, an imidazolidinyl group, a pyrazolidinyl group and a dioxolanyl group; and a 6-membered saturated heterocyclyl group such as a piperidyl group, a piperazinyl group, a morpholinyl group, a tetrahydropyranyl group, a dioxolanyl group and a dioxanyl group.

Examples of the 5- to 6-membered partially unsaturated heterocyclic group include a 5-membered partially unsaturated heterocyclic group such as a pyrrolinyl group, a dihydrofuranyl group, an imidazolinyl group, a pyrazolinyl group and an oxazolinyl group; and a 6-membered partially unsaturated heterocyclic group such as a dihydropyranyl group.

Examples of the 5- to 6-membered heteroaryl group include a 5-membered heteroaryl group such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group; and a 6-membered heteroaryl group such as a pyridyl group, a pyrazinyl group, a pyrimidinyl group, a pyridazinyl group and a triazinyl group.

[$R^1$]

In the formula (I), $R^1$ represents a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{3-6}$ cycloalkyl group,

or a 5- to 6-membered cyclic ether group.

The "$C_{1-6}$ alkyl group" represented by $R^1$ may be linear or branched. Examples of the "$C_{1-6}$ alkyl group" represented by $R^1$ include a methyl group, an ethyl group, an n-propyl group, an n-butyl group, an n-pentyl group, an n-hexyl group, an i-propyl group, an i-butyl group an s-butyl group, a t-butyl group, an i-pentyl group, a neopentyl group, a 2-methylbutyl group and an i-hexyl group.

Examples of the "$C_{2-6}$ alkenyl group" represented by $R^1$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group and a 5-hexenyl group.

Examples of the "$C_{2-6}$ alkynyl group" represented by $R^1$ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group.

The substituent on the "$C_{1-6}$ alkyl group", "$C_{2-6}$ alkenyl group", or "$C_{2-6}$ alkynyl group" represented by $R^1$ is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; or a cyano group.

Examples of the "$C_{3-6}$ cycloalkyl group" represented by $R^1$ include a cyclopropyl group, a cyclobutyl group, a cyclopentyl group and a cyclohexyl group.

Examples of the "5- to 6-membered cyclic ether group" represented by $R^1$ include a tetrahydrofuranyl group and a tetrahydropyranyl group.

The substituent on the "$C_{3-6}$ cycloalkyl group" represented by $R^1$ is preferably a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a difluoromethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; or a cyano group.

$R^1$ is preferably a substituted or unsubstituted $C_{1-6}$ alkyl group or a 5- to 6-membered cyclic ether group, and more preferably a substituted or unsubstituted $C_{1-6}$ alkyl group.

The substituent on the "$C_{1-6}$ alkyl group" represented by $R^1$ is preferably a halogeno group, a $C_{1-6}$ alkoxy group, a $C_{1-6}$ haloalkoxy group, a $C_{1-6}$ alkylthio group, a $C_{1-6}$ alkylsulfinyl group, a $C_{1-6}$ alkylsulfonyl group or a $C_{3-6}$ cycloalkyl group.

[R2]

In the formula (I), $R^2$ represents a substituted or unsubstituted $C_{1-6}$ alkyl group, a substituted or unsubstituted $C_{2-6}$ alkenyl group, or a substituted or unsubstituted $C_{2-6}$ alkynyl group.

Specific examples of the "unsubstituted $C_{1-6}$ alkyl group", "substituted or unsubstituted $C_{2-6}$ alkenyl group", or "substituted or unsubstituted $C_{2-6}$ alkynyl group" represented by $R^2$ include the same as those exemplified for $R^1$.

The substituent on the "$C_{1-6}$ alkyl group" represented by $R^2$ is preferably a halogeno group such as a fluoro group, a chloro group, a bromo group and an iodo group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ alkoxy $C_{1-6}$ alkoxy group such as a methoxyethoxy group; a $C_{1-6}$ haloalkoxy group such as a 2,3-dichlorobutoxy group, a trifluoromethoxy group and a 2,2,2-trifluoroethoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl

group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; a $C_{1-6}$ alkyl group substituted, halogeno group substituted, $C_{1-6}$ haloalkyl group substituted or $C_{1-6}$ haloalkoxy group substituted phenoxy group; a 5-membered heteroaryl group; a $C_{1-6}$ alkyl group substituted, halogeno group substituted, $C_{1-6}$ haloalkyl group substituted or $C_{1-6}$ haloalkoxy group substituted 5-membered heteroaryl group; a $C_{1-6}$ alkylcarbonyl group such as an acetyl group; a benzoyl group; a $C_{1-6}$ alkoxycarbonyl group such as a methoxycarbonyl group; a $C_{1-6}$ alkylcarboxamide group such as an acetamide group; a trimethylsilyl group or a cyano group.

The "5-membered heteroaryl group" mentioned as one of the substituents on the "$C_{1-6}$ alkyl group" represented by $R^2$ is a group of a 5-membered aromatic ring containing 1 to 4 hetero atoms selected from the group consisting of a nitrogen atom, an oxygen atom and a sulfur atom as constituent atoms of the ring.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

$R^2$ is preferably a substituted or unsubstituted $C_{1-6}$ alkyl group.

[$R^3$]

In the formula (I), $R^3$ represents a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{3-6}$ cycloalkyl group or a substituted or unsubstituted phenyl group.

Specific examples of these groups represented by $R^3$ include the same as those exemplified for $R^1$.

Examples of the "$C_{1-6}$ alkoxy group" represented by $R^3$ include a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group and an i-hexyloxy group.

The substituent on the "$C_{1-6}$ alkoxy group" represented by $R^3$ is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; or a cyano group.

The substituent on the "phenyl group" represented by $R^3$ is preferably a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a difluoromethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; or a cyano group.

$R^3$ is preferably a hydrogen atom.

[Q]

In the formula (I), Q represents a substituted or unsubstituted 5- to 10-membered heterocyclyl group.

The 5- to 10-membered heterocyclyl group contains 1 to 4 hetero atoms selected from the group consisting of a nitrogen atom, an oxygen atom and a sulfur atom as constituent atoms of the ring. When there are two or more hetero atoms, they may be the same or different. The heterocyclyl group may be either monocyclic or polycyclic. As long as the polycyclic heterocyclyl group includes at least one heterocyclic ring, the remaining ring may be any of a saturated alicyclic ring, an unsaturated alicyclic ring or an aromatic ring. Examples of the 5- to 10-membered heterocyclyl group include a 5-to 10-membered saturated heterocyclyl group, a 5- to 10-membered partially unsaturated heterocyclyl group and a 5- to 10-membered heteroaryl group, and preferred examples thereof include a 5-membered saturated heterocyclyl

group, a 5-membered partially unsaturated heterocyclyl group, a 5-membered heteroaryl group, a 6-membered saturated heterocyclyl group, a 6-membered partially unsaturated heterocyclyl group, a 6-membered heteroaryl group, a 9-membered heteroaryl group, and a 10-membered heteroaryl group.

Examples of the 5-membered saturated heterocyclyl group include a pyrrolidinyl group, a tetrahydrofuranyl group, a thiazolidinyl group, an imidazolidinyl group, a pyrazolidinyl group, and a dioxolanyl group.

Examples of the 5-membered partially unsaturated heterocyclic group include a pyrrolinyl group, a dihydrofuranyl group, an imidazolinyl group, a pyrazolinyl group, and an oxazolinyl group.

Examples of the 5-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group.

Examples of the 6-membered saturated heterocyclyl group include a piperidyl group, a piperazinyl group, a morpholinyl group, a tetrahydropyranyl group, a dioxolanyl group, and a dioxanyl group.

Examples of the 6-membered partially unsaturated heterocyclic group include a dihydropyranyl group.

Examples of the 6-membered heteroaryl group include a pyridyl group, a pyrazinyl group, a pyrimidinyl group, a pyridazinyl group and a triazinyl group.

Examples of the 9-membered heteroaryl group include an indolyl group, an isoindolyl group, a benzofuranyl group, a benzothienyl group, an indazolyl group, a benzimidazolyl group, a benzoxazolyl group, a benzisoxazolyl group, a benzothiazolyl group and a benzisothiazolyl group.

Examples of the 10-membered heteroaryl group include a quinolinyl group, an isoquinolinyl group, a cinnolinyl group, a phthalazinyl group, a quinazolinyl group, a quinoxalinyl group, and a naphthyridinyl group.

Q is preferably a substituted or unsubstituted 5- to 10-membered heteroaryl group, and more preferably a substituted or unsubstituted 5-membered heteroaryl group, a substituted or unsubstituted 6-membered heteroaryl group, a substituted or unsubstituted 9-membered heteroaryl group or a substituted or unsubstituted 10-membered heteroaryl group.

[X]

The substituent on the "5- to 10-membered heterocyclyl group" represented by Q (sometimes referred to as substituent (X)) is at least one selected from the group consisting of a halogeno group, a substituted or unsubstituted $C_{1-6}$ alkyl group, a substituted or unsubstituted $C_{2-6}$ alkenyl group, a substituted or unsubstituted $C_{2-6}$ alkynyl group, a hydroxyl group, a substituted or unsubstituted $C_{1-6}$ alkoxy group, a substituted or unsubstituted $C_{2-6}$ alkenyloxy group, a substituted or unsubstituted $C_{2-6}$ alkynyloxy group, a substituted or unsubstituted $C_{1-6}$ alkylthio group, a substituted or unsubstituted $C_{1-6}$ alkylsulfinyl group, a substituted or unsubstituted $C_{1-6}$ alkylsulfonyl group, a substituted or unsubstituted $C_{3-6}$ cycloalkyl group, a substituted or unsubstituted $C_{3-6}$ cycloalkyloxy group, a substituted or unsubstituted phenyl group, a phenoxy group, a substituted or unsubstituted 5- to 6-membered heterocyclyl group, a substituted or unsubstituted 5- to 6-membered heterocyclyloxy group, a substituted or unsubstituted phenylsulfonyl group, a group represented by R-CO-, a group represented by RO-CO-, a group represented by R-CONR$^a$-, a group represented by RNH-CO-, a group represented by $R_2N$-CO-, a group represented by RO-CO-NR$^a$-, a group represented by RNH-CO-NH-, a group represented by $R_2N$-CO-NH-, a group represented by RNH-CO-CO-NH-, a group represented by $R_2N$-CO-CO-NH-, a group represented by R-$S(O)_2$-NH-, a group represented by $R_2N$-$S(O)_2$-, a group represented by RO-N=C(R$^c$)-, a nitro group, a cyano group and an oxo group.

Here, each R independently represents a substituted or unsubstituted $C_{1-6}$ alkyl group or a substituted or unsubstituted $C_{3-6}$ cycloalkyl group.

Each R$^a$ independently represents a hydrogen atom, a substituted or unsubstituted $C_{1-6}$ alkyl group, or a substituted or unsubstituted $C_{1-6}$ alkoxy group.

R$^c$ represents a hydrogen atom or a substituted or unsubstituted $C_{1-6}$ alkyl group.

Further, in the above group represented by $R_2N$-CO-, the group represented by $R_2N$-CO-NH-, the group represented by $R_2N$-CO-CO-NH-, or the group represented by $R_2N$-$S(O)_2$-, the two R groups may be bonded with each other to form a 4- to 6-membered ring together with a nitrogen atom to which they are bonded.

Examples of the "halogeno group" represented by X include a fluoro group, a chloro group, a bromo group and an iodo group.

The "$C_{1-6}$ alkyl group" represented by X may be linear or branched. Examples of the "$C_{1-6}$ alkyl group" represented by X include a methyl group, an ethyl group, an n-propyl group, an n-butyl group, an n-pentyl group, an n-hexyl group, an i-propyl group, an i-butyl group, an s-butyl group, a t-butyl group, an i-pentyl group, a neopentyl group, a 2-methylbutyl group and an i-hexyl group.

Examples of the "$C_{2-6}$ alkenyl group" represented by X include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a

2-methyl-2-butenyl group, a 1-hexenyl group, a 2-hexenyl group, a 3-hexenyl group, a 4-hexenyl group and a 5-hexenyl group.

Examples of the "$C_{2-6}$ alkynyl group" represented by X include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group and a 1,1-dimethyl-2-butynyl group.

Examples of the "$C_{1-6}$ alkoxy group" represented by X include a methoxy group, an ethoxy group, an n-propoxy group, an n-butoxy group, an n-pentyloxy group, an n-hexyloxy group, an i-propoxy group, an i-butoxy group, an s-butoxy group, a t-butoxy group and an i-hexyloxy group.

Examples of the "$C_{2-6}$ alkenyloxy group" represented by X include a vinyloxy group, an allyloxy group, a propenyloxy group and a butenyloxy group.

Examples of the "$C_{2-6}$ alkynyloxy group" represented by X include an ethynyloxy group and a propargyloxy group.

Examples of the "$C_{1-6}$ alkylthio group" represented by X include a methylthio group, an ethylthio group, an n-propylthio group, an n-butylthio group, an n-pentylthio group, an n-hexylthio group and an i-propylthio group.

Examples of the "$C_{1-6}$ alkylsulfinyl group" represented by X include a methylsulfinyl group, an ethylsulfinyl group and a t-butylsulfinyl group.

Examples of the "$C_{1-6}$ alkylsulfonyl group" represented by X include a methylsulfonyl group, an ethylsulfonyl group and a t-butylsulfonyl group.

The substituent on the "$C_{1-6}$ alkyl group" or "$C_{1-6}$ alkoxy group" represented by X is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ alkoxy $C_{1-6}$ alkoxy group such as a methoxyethoxy group; a $C_{3-6}$ cycloalkyl $C_{1-6}$ alkoxy group such as a cyclopropylmethoxy group; a $C_{1-6}$ haloalkoxy group such as a 2,3-dichlorobutoxy group, a trifluoromethoxy group, a 2,2,2-trifluoroethoxy group and a 3,3,3-trifluoropropoxy group; a $C_{1-6}$ alkylthio group such as a methylthio group, an ethylthio group, an n-propylthio group, an i-propylthio group, an n-butylthio group, an i-butylthio group, an s-butylthio group and a t-butylthio group; a $C_{1-6}$ alkylsulfinyl group such as a methylsulfinyl group, an ethylsulfinyl group and a t-butylsulfinyl group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; a morpholinyl group; a 5-membered heteroaryl group such as a triazolyl group; a $C_{1-6}$ alkyl group substituted, halogeno group substituted, $C_{1-6}$ haloalkyl group substituted or $C_{1-6}$ haloalkoxy group substituted 5-membered heteroaryl group; a $C_{1-6}$ alkyl group substituted aminocarbonyl group such as a methylaminocarbonyl group and a dimethylaminocarbonyl group; or a cyano group.

The substituent on the "$C_{2-6}$ alkenyl group", "$C_{2-6}$ alkynyl group", "$C_{2-6}$ alkynyloxy group", "$C_{1-6}$ alkylthio group", "$C_{1-6}$ alkylsulfinyl group", or "$C_{1-6}$ alkylsulfonyl group" represented by X is preferably a halogeno group such as a fluoro group, a chloro group, a bromo group and an iodo group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; a $C_{1-6}$ alkylsulfonyl group such as a methylsulfonyl group, an ethylsulfonyl group and a t-butylsulfonyl group; a $C_{3-6}$ cycloalkyl group such as a cyclopropyl group, a cyclobutyl group, a cyclopentyl group and a cyclohexyl group; a phenyl group; a $C_{1-6}$ alkyl group substituted, halogeno group substituted, $C_{1-6}$ haloalkyl group substituted or $C_{1-6}$ haloalkoxy group substituted phenyl group such as a 4-methylphenyl group, a 4-chlorophenyl group, a 4-trifluoromethylphenyl group and a 4-trifluoromethoxyphenyl group; or a cyano group.

Examples of the "$C_{3-6}$ cycloalkyl group" represented by X include a cyclopropyl group, a cyclobutyl group, a cyclopentyl group and a cyclohexyl group.

Examples of the "$C_{3-6}$ cycloalkyloxy group" represented by X include a cyclopropyloxy group, a cyclobutyloxy group, a cyclopentyloxy group and a cyclohexyloxy group.

The "5- to 6-membered heterocyclyl group" represented by X is a 5- or 6-membered ring group containing 1 to 4 hetero atoms selected from the group consisting of a nitrogen atom, an oxygen atom and a sulfur atom as constituent atoms of the ring. When there are two or more hetero atoms, they may be the same or different. Examples of the "5- to 6-membered heterocyclyl group" include a 5- to 6-membered saturated heterocyclyl group, a 5- to 6-membered partially unsaturated heterocyclyl group, and a 5-to 6-membered heteroaryl group.

Examples of the 5- to 6-membered saturated heterocyclyl group include a 5-membered saturated heterocyclyl group such as a pyrrolidinyl group, a tetrahydrofuranyl group, a thiazolidinyl group, an imidazolidinyl group, a pyrazolidinyl group and a dioxolanyl group; and a 6-membered saturated heterocyclyl group such as a piperidyl group, a piperazinyl group, a morpholinyl group, a tetrahydropyranyl group, a dioxolanyl group and a dioxanyl group.

Examples of the 5- to 6-membered partially unsaturated heterocyclic group include a 5-membered partially

unsaturated heterocyclic group such as a pyrrolinyl group, a dihydrofuranyl group, an imidazolinyl group, a pyrazolinyl group and an oxazolinyl group; and a 6-membered partially unsaturated heterocyclic group such as a dihydropyranyl group.

Examples of the 5- to 6-membered heteroaryl group include a 5-membered heteroaryl group such as a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group and a tetrazolyl group; and a 6-membered heteroaryl group such as a pyridyl group, a pyrazinyl group, a pyrimidinyl group, a pyridazinyl group and a triazinyl group.

The "5- to 6-membered heterocyclyloxy group" represented by X has a structure in which a 5- to 6-membered heterocyclyl group and an oxy group are bonded. Specific examples thereof include a thiazolyloxy group and a pyridyloxy group.

The substituent on the "$C_{3-6}$ cycloalkyl group", "$C_{3-6}$ cycloalkyloxy group", "phenyl group", "phenoxy group", "5- to 6-membered heterocyclyl group", "5- to 6-membered heterocyclyloxy group" or "phenylsulfonyl group" represented by X is preferably a halogeno group such as a fluoro group, a chloro group, a bromo group and an iodo group; a $C_{1-6}$ alkyl group such as a methyl group, an ethyl group, an n-propyl group, an i-propyl group, an n-butyl group, an s-butyl group, an i-butyl group, a t-butyl group, an n-pentyl group and an n-hexyl group; a $C_{1-6}$ haloalkyl group such as a difluoromethyl group, a trifluoromethyl group, a 1,2-dichloro-n-propyl group and a 1-fluoro-n-butyl group; a hydroxyl group; a $C_{1-6}$ alkoxy group such as a methoxy group, an ethoxy group, an n-propoxy group, an i-propoxy group, an n-butoxy group, an s-butoxy group, an i-butoxy group and a t-butoxy group; a $C_{1-6}$ haloalkoxy group such as a 2,3-dichlorobutoxy group, a trifluoromethoxy group and a 2,2,2-trifluoroethoxy group; an oxo group; or a cyano group.

Specific examples of these groups represented by R, $R^a$, or $R^c$ include the same as those exemplified for X.

Examples of the "group represented by R-CO-" represented by X include an acetyl group and a cyclopropylcarbonyl group.

Examples of the "group represented by RO-CO-" represented by X include a methoxycarbonyl group.

Examples of the "group represented by R-CONR$^a$-" represented by X include an acetamide group and a cyclopropanecarboxamide group.

Examples of the "group represented by RNH-CO-" represented by X include a methylaminocarbonyl group.

Examples of the "group represented by $R_2$N-CO-" represented by X include a dimethylaminocarbonyl group.

Here, the two R groups may be bonded with each other to form a 4- to 6-membered ring together with a nitrogen atom to which they are bonded, and examples of the 4- to 6-membered ring to be formed include an azetidine ring, a pyrrolidine ring, a piperidine ring, a piperazine ring, and a morpholine ring.

Examples of the "group represented by $R_2$N-CO-" after forming a 4- to 6-membered ring include an azetidine-1-carbonyl group, a pyrrolidine-1-carbonyl group and a morpholine-4-carbonyl group.

Examples of the "group represented by RO-CO-NR$^a$-" represented by X include a (t-butoxycarbonyl) amino group and a methoxy (t-butoxycarbonyl) amino group.

Examples of the "group represented by RNH-CO-NH-" represented by X include a methylureido group.

Examples of the "group represented by $R_2$N-CO-NH-" represented by X include a 3,3-dimethylureido group.

Here, the two R groups may be bonded with each other to form a 4- to 6-membered ring together with a nitrogen atom to which they are bonded, and specific examples of the 4- to 6-membered ring to be formed include the same as those exemplified above for the "group represented by $R_2$N-CO-".

Examples of the "group represented by $R_2$N-CO-NH-" after forming a 4- to 6-membered ring include an azetidine-1-carboxamide group, a pyrrolidine-1-carboxamide group and a morpholine-4-carboxamide group.

Examples of the "group represented by RNH-CO-CO-NH-" represented by X include a methylaminocarbonylcarboxamide group.

Examples of the "group represented by $R_2$N-CO-CO-NH-" represented by X include a dimethylaminocarbonylcarboxamide group.

Here, the two R groups may be bonded with each other to form a 4- to 6-membered ring together with a nitrogen atom to which they are bonded, and specific examples of the 4- to 6-membered ring to be formed include the same as those exemplified above for the "group represented by $R_2$N-CO-".

Examples of the "group represented by $R_2$N-CO-NH-" after forming a 4- to 6-membered ring include an azetidine-1-carbonylcarboxamide group, a pyrrolidine-1-carbonylcarboxamide group and a morpholine-4-carbonylcarboxamide.

Examples of the "group represented by R-S(O)$_2$-NH-" represented by X include a methylsulfonamide group.

Examples of the "group represented by $R_2$N-S(O)$_2$-" represented by X include a dimethylaminosulfonyl group.

Here, the two R groups may be bonded with each other to form a 4- to 6-membered ring together with a nitrogen atom to which they are bonded, and specific examples of the 4- to 6-membered ring to be formed include the same as those exemplified above for the "group represented by $R_2$N-CO-".

Examples of the "group represented by $R_2$N-S(O)$_2$-" after forming a 4- to 6-membered ring include an azetidine-1-sulfonyl group, a pyrrolidine-1-sulfonyl group and a morpholinosulfonyl group.

Examples of the "group represented by RO-N=C($R^c$)-" represented by X include a (methoxyimino) methyl group and a 1-(methoxyimino) ethyl group.

Q is a "5- to 10-membered heterocyclyl group substituted with an oxo group" when X is an oxo group. Examples of the 5- to 10-membered heterocyclyl group substituted with an oxo group include a 2-oxo-pyrrolidin-3-yl group, a 2-oxo-1,2-dihydropyridin-3-yl group, a 6-oxo-1,6-dihydropyridin-2-yl group, a 6-oxo-1,6-dihydropyrimidin-5-yl group, a 3-oxo-3,4-dihydropyrazin-2-yl group, a 2,4-dioxo-1,2,3,4-tetrahydropyrimidin-5-yl group, a 3,5-dioxo-2,3,4,5-tetrahydro-1,2,4-triazin-6-yl group, a 2-oxo-1,2-dihydroquinolin-3-yl group, a 3-oxo-3,4-dihydroquinoxalin-2-yl group and a 2-oxo-1,2-dihydro-1,7-naphthalidin-3-yl group.

X is preferably a halogeno group, a substituted or unsubstituted $C_{1-6}$ alkyl group, a substituted or unsubstituted $C_{1-6}$ alkoxy group, a substituted or unsubstituted $C_{1-6}$ alkylsulfonyl group, a substituted or unsubstituted $C_{3-6}$ cycloalkyl group, a substituted or unsubstituted phenyl group, a substituted or unsubstituted 5- to 6-membered heterocyclyl group, $R_2N-S(O)_2-$, or an oxo group.

The compound (I) is not particularly limited depending on the production method thereof. Further, the salt of the compound (I) can be obtained from the compound (I) by a known method. The compound (I) can be produced, for example, by the method described in Examples and the like using the compound obtained by the production method described in Patent Document 1.

The synthesis of the compound (I) can be carried out by, for example, Scheme 1.

(Scheme 1)

[Chemical Formula 3]

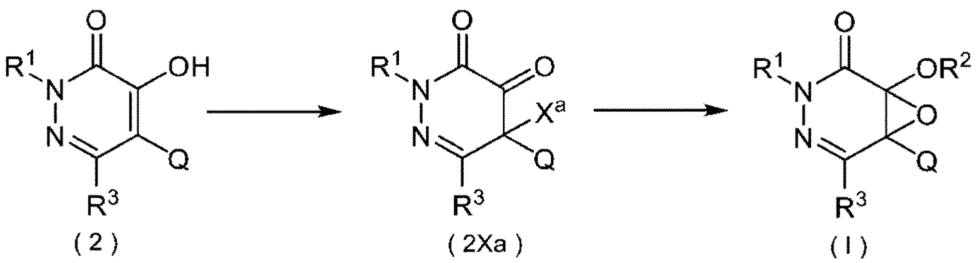

More specifically, the compound (I) is synthesized by reacting a compound represented by a formula (2) (hereinafter, may be referred to as compound (2)) with a halogenating agent to construct an $\alpha$-haloketone structure in the molecule and obtaining a compound represented by a formula (2Xa) (hereinafter, may be referred to as compound (2Xa)), and subsequently reacting the compound (2Xa) with an alkoxide such as $R^2ONa$ (corresponds to sodium methoxide when $R^2$ is a methyl group). $X^a$ in the formula (2Xa) represents a halogeno group such as a chloro group and a bromo group. The compound (2Xa) may be unstable, and it is preferable to carry out the subsequent reaction without isolation. The symbols in the formulas (2) and (2Xa) have the same meanings as those defined in the formula (I).

The synthesis of the compound (2) can be carried out by, for example, Scheme 2.

(Scheme 2)

[Chemical Formula 4]

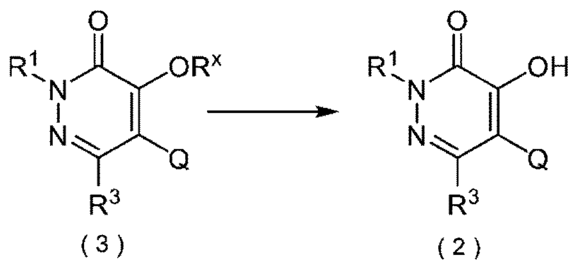

More specifically, the compound (2) can be obtained by heating a compound represented by a formula (3)

(hereinafter, may be referred to as compound (3)) together with morpholine. The symbols in the formula (3) have the same meanings as those defined in the formula (I). $R^x$ represents a lower alkyl group, such as a methyl group. Hereinafter, $R^x$ has the same meaning as defined above.

The synthesis of the compound (3) can be carried out by, for example, Scheme 3.

(Scheme 3)

[Chemical Formula 5]

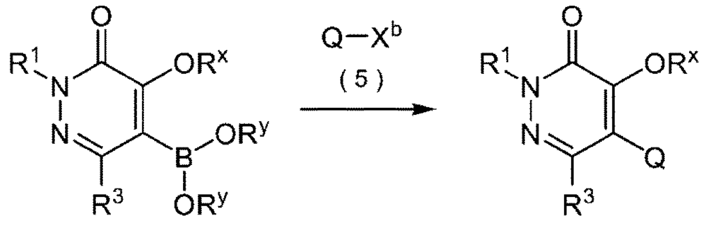

(4) (3)

More specifically, the compound (3) can be obtained by condensation of a compound represented by a formula (4) (hereinafter, may be referred to as the compound (4)) with a compound represented by a formula (5). The condensation is preferably carried out in the presence of a base (for example, an inorganic base such as potassium phosphate or cesium fluoride), a metal catalyst (for example, a palladium catalyst such as $Pd(OAc)_2$), and according to circumstances, a ligand (for example, a phosphine ligand). The symbols in the formula (4) have the same meanings as those defined in the formula (I). $R^y$ represents a lower alkyl group, such as a methyl group, an ethyl group and the like. Further, $R^y$ groups may be bonded to each other to form a 1,3,2-dioxaborolane ring. Q in the formula (5) has the same meaning as Q in the formula (I). $X^b$ represents a halogeno group. In Q in the formula (5), the substituent on the 5- to 10-membered heterocyclyl group may be appropriately converted even after the reaction.

The metal catalyst and ligand can be added to the reaction system in the form of a complex (for example, a palladium / phosphine complex such as bis (triphenylphosphine) palladium dichloride or a [1,1-bis (diphenylphosphino) ferrocene] palladium dichloride dichloromethane adduct).

The synthesis of the compound (4) can be carried out by, for example, Scheme 4.

(Scheme 4)

[Chemical Formula 6]

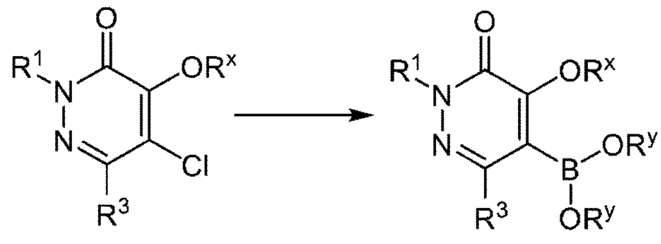

(6) (4)

More specifically, the compound (4) can be obtained by reacting a compound represented by a formula (6) (hereinafter, may be referred to as compound (6)) with boronic acid or an ester of boronic acid, such as bis (pinacolato) diboron, in the presence of a base (for example, an inorganic base such as potassium phosphate or cesium fluoride), a metal catalyst (for example, a palladium catalyst such as $Pd_2(dba)_3$ and ($Pd(OAc)_2$)) and, according to circumstances, a ligand (for example, a phosphine ligand). The symbols in the formula (6) have the same meanings as those defined in the formula (I).

The metal catalyst and ligand can be added to the reaction system in the form of a complex (for example, a palladium / phosphine complex such as bis (triphenylphosphine) palladium dichloride or a [1,1-bis (diphenylphosphino) ferrocene] palladium dichloride dichloromethane adduct).

The synthesis of the compound (6) can be carried out by, for example, Scheme 5.

(Scheme 5)

[Chemical Formula 7]

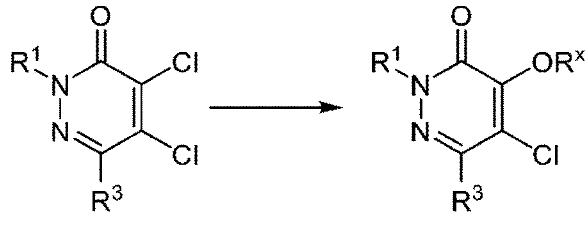

(7) (6)

The compound (6) can be obtained by reacting a compound represented by a formula (7) (hereinafter, may be referred to as compound (7)) with a metal alkoxide, for example, sodium methoxide. The compound (7) can be synthesized by a known method. The symbols in the formula (7) have the same meanings as those defined in the formula (I).

The synthesis of the compound (3) can also be carried out by Scheme 3A.

(Scheme 3A)

[Chemical Formula 8]

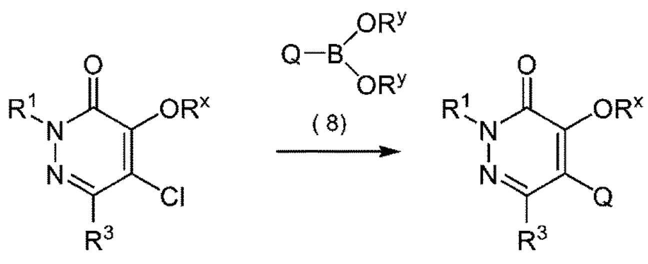

(6) (3)

More specifically, the compound (3) can be obtained by condensation of the compound represented by the formula (6) with a compound represented by a formula (8). Q in the formula (8) has the same meaning as Q in the formula (I). $R^y$ represents a lower alkyl group, such as a methyl group, an ethyl group and the like. Further, the two $R^y$ groups may be bonded to each other to form a 1,3,2-dioxaborolane ring. This condensation is preferably carried out in the presence of a base (for example, an inorganic base such as potassium phosphate or cesium fluoride), a metal catalyst (for example, a palladium catalyst such as $Pd(OAc)_2$), and according to circumstances, a ligand (for example, a phosphine ligand).

The metal catalyst and ligand can be added to the reaction system in the form of a complex (for example, a palladium / phosphine complex such as bis (triphenylphosphine) palladium dichloride or a [1,1-bis (diphenylphosphino) ferrocene] palladium dichloride dichloromethane adduct).

In Q in the formula (8), the substituent on the 5- to 10-membered heterocyclyl group may be appropriately converted even after the reaction.

The compound of the present invention exhibits high herbicidal activity in both methods of soil treatment and foliage treatment under upland farming conditions. The compound of the present invention is effective against various field weeds and may show selectivity for crops such as maize and wheat. In addition, the compound of the present invention may exhibit plant growth regulating effects such as growth inhibitory effects on useful plants such as crops, ornamental plants and fruit trees. Further, the compound of the present invention has excellent herbicidal effects on paddy weeds and may show selectivity for rice.

The herbicide of the present invention contains at least one selected from the group consisting of the compound (I) and a salt of the compound (I) as an active ingredient.

The herbicide of the present invention exhibits high herbicidal activity in both methods of soil treatment and foliage treatment under upland farming conditions. In addition, the herbicide of the present invention has excellent herbicidal effects on paddy weeds such as Echinochloa spp., Cyperus difforis, Sagittaria trifolia and Schoenoplectiella hotarui, and may show selectivity for rice. Furthermore, the herbicide of the present invention can also be applied for the control of weeds in places such as orchards, lawns, track ends and vacant sites.

Useful plants for which the herbicide of the present invention can be used include grains, for example, barley and wheat, and crops such as cotton, rapeseed, sunflower, maize, rice, soybean, sugar beet, sugar cane and lawn. Crops may also include trees such as fruit trees, palm trees, coconut trees or other nuts, and also include vines such as grapes, fruit shrubs, fruit plants and vegetables.

Examples of upland field weeds to be controlled include the following weeds.

(A) Monocotyledonous weeds

(1) Weeds of the family Cyperaceae

Weeds of the genus Cyperus such as Cyperus esculentus, Cyperus iria, Cyperus microiria and Cyperus rotundus.

(2) Weeds of the family Poaceae

Weeds of the genus Alopecurus such as Alopecurus aequalis and Alopecurus myosuroides;
Weeds of the genus Apera such as Apera spica-venti;
Weeds of the genus Avena such as Avena sativa;
Weeds of the genus Bromus such as Bromus japonicus and Bromus sterilis;
Weeds of the genus Digitaria such as Digitaria ciliaris and Digitaria sanguinalis;
Weeds of the genus Echinochloa such as Echinochloa crus-galli;
Weeds of the genus Eleusine such as Eleusine indica;
Weeds of the genus Lolium such as Lolium multiflorum Lam.;
Weeds of the genus Panicum such as Panicum dichotomiflorum;
Weeds of the genus Poa such as Poa annua;
Weeds of the genus Setaria such as Setaria faberi, Setaria pumila and Setaria viridis;
Weeds of the genus Sorghum such as Sorghum bicolor; and
Weeds of the genus Urochloa such as Urochloa platyphylla.

(B) Dicotyledonous weeds

(1) Weeds of the family Amaranthaceae

Weeds of the genus Amaranthus such as Amaranthus blitum, Amaranthus palmeri, Amaranthus retroflexus and Amaranthus rudis;
Weeds of the genus Chenopodium such as Chenopodium album;
Weeds of the genus Bassia such as Bassia scoparia.

(2) Weeds of the family Asteraceae

Weeds of the genus Ambrosia such as Ambrosia artemisiifolia and Ambrosia trifida;
Weeds of the genus Conyza such as Conyza canadensis and Conyza sumatrensis;
Weeds of the genus Erigeron such as Erigeron annuus;
Weeds of the genus Matricaria such as Matricaria inodora and Matricaria recutita;
Weeds of the genus Xanthium such as Xanthium occidentale.

(3) Weeds of the family Caryophyllaceae

Weeds of the genus Sagina such as Sagina japonica;
Weeds of the genus Stellaria such as Stellaria media.

(4) Weeds of the family Convolvulaceae

Weeds of the genus Calystegia such as Calystegia japonica;
Weeds of the genus Ipomoea such as Ipomoea coccinea, Ipomoea hederacea, Ipomoea lacunosa and Ipomoea triloba.

(5) Weeds of the family Lamiaceae

Weeds of the genus Lamium such as Lamium album var. barbatum, Lamium amplexicaule and Lamium purpureum.

(6) Weeds of the family Malvaceae

Weeds of the genus Abutilon such as Abutilon theophrasti;
Weeds of the genus Sida such as Sida spinosa.

(7) Weeds of the family Plantaginaceae

Weeds of the genus Veronica such as Veronica persica.

(8) Weeds of the family Polygonaceae

Weeds of the genus Fallopia such as Fallopia convolvulus.
Weeds of the genus Persicaria such as Persicaria lapathifolia and Persicaria longiseta.

(9) Weeds of the family Rubiaceae

Weeds of the genus Galium such as Galium spurium var. echinospermon.
Examples of paddy weeds to be controlled include the following weeds.

(A) Monocotyledonous weeds

(1) Weeds of the family Alismataceae

Weeds of the genus Sagittaria such as Sagittaria pygmaea Miq. and Sagittaria trifolia.

(2) Weeds of the family Cyperaceae

Weeds of the genus Cyperus such as Cyperus serotinus and Cyperus difforis;
Weeds of the genus Eleocharis such as Eleocharis kuroguwai Ohwi;
Weeds of the genus Schoenoplectiella such as Schoenoplectiella hotarui and Schoenoplectiella juncoides Roxb.
Weeds of the genus Scirpus such as Scirpus maritimus and Scirpus nipponicus.

(3) Weeds of the family Poaceae

Weeds of the genus Echinochloa such as Echinochloa oryzoides and Echinochloa crus-galli;
Weeds of the genus Leersia such as Leersia japonica;
Weeds of the genus Paspalum such as Paspalum distichum.

(4) Weeds of the family Pontederiaceae

Weeds of the genus Monochoria such as Monochoria korsakowii and Monochoria vaginalis var. plantaginea.

(B) Dicotyledonous weeds

(1) Weeds of the family Apiaceae

Weeds of the genus Oenanthe such as Oenanthe javanica.

(2) Weeds of the family Elatinaceae

Weeds of the genus Elatine such as Elatine triandra.

(3) Weeds of the family Linderniaceae

Weeds of the genus Lindernia such as Lindernia dubia subsp. major, Lindernia dubia subsp. dubia and Lindernia procumbens.

(4) Weeds of the family Lythraceae

Weeds of the genus Rotala such as Rotala indica var. uliginosa.

The herbicide of the present invention may consist only of the compound of the present invention, or may be formulated into a dosage form generally adopted as an agricultural chemical, for example, a wettable powder, a granule, a powder, an emulsion, a water soluble powder, a suspension, a flowable or the like.

For formulation, the herbicide of the present invention may contain a known agrochemically acceptable additive or carrier. As the additive or carrier, either a solid form or a liquid form can be used.

For solid dosage forms, vegetable powders such as soy flour and wheat flour, fine mineral powders such as diatomaceous earth, apatite, gypsum, talc, bentonite, pyrophyllite and clay, and solid carriers of organic and inorganic compounds such as sodium benzoate, urea and mirabilite can be used.

For liquid dosage forms, petroleum fractions such as kerosine, xylene and solvent naphtha, and liquid carriers such as cyclohexane, cyclohexanone, dimethylformamide, dimethyl sulfoxide, alcohols, acetone, trichloroethylene, methyl isobutyl ketone, mineral oil, vegetable oil and water can be used.

In formulation, a surfactant can be added as needed. Examples of the surfactant include nonionic surfactants such as alkylphenyl ethers to which polyoxyethylene is added, alkyl ethers to which polyoxyethylene is added, higher fatty acid esters to which polyoxyethylene is added, sorbitan higher fatty acid esters to which polyoxyethylene is added, and tristyrylphenyl ethers to which polyoxyethylene is added, sulfuric acid ester salts of alkylphenyl ethers to which polyoxyethylene is added, alkylnaphthalene sulfonate, polycarboxylate, lignin sulfonate, formaldehyde condensates of alkylnaphthalene sulfonate, and isobutylene-maleic anhydride copolymers.

In the herbicide of the present invention, the concentration of the active ingredient can be appropriately set according to the dosage form. For example, the concentration of the active ingredient in a wettable powder is preferably from 5 to 90% by weight, and more preferably from 10 to 85% by weight. The concentration of the active ingredient in an emulsion is preferably from 3 to 70% by weight, and more preferably from 5 to 60% by weight. The concentration of the active ingredient in a granule is preferably from 0.01 to 50% by weight, and more preferably from 0.05 to 40% by weight.

The wettable powder or emulsion obtained in this manner can be used as a suspension or emulsion by diluting with water to a predetermined concentration, and the granules can be directly sprayed on or mixed with the soil before or after germination of weeds. When applying the herbicide of the present invention to a farm field, an appropriate amount of 0.1 g or more of the active ingredient can be applied per hectare.

In addition, the herbicide of the present invention may contain a known fungicide, fungicidal active ingredient, insecticide, insecticidal active ingredient, acaricide, acaricidal active ingredient, herbicide, herbicidal active ingredient,

plant growth regulator, fertilizer, phytotoxicity reducing agent (safener) or the like. Alternatively, when using the herbicide of the present invention, a known fungicide, fungicidal active ingredient, insecticide, insecticidal active ingredient, acaricide, acaricidal active ingredient, herbicide, herbicidal active ingredient, plant growth regulator, fertilizer, phytotoxicity reducing agent (safener) or the like may be mixed. In particular, by using the herbicide of the present invention in combination with a conventional herbicide, it is possible to reduce the amount of the drug used. Further, in addition to labor saving, even higher effects can also be expected due to the synergistic action of the mixed drug. In that case, a combination with a plurality of known herbicides is also possible.

The conventional herbicidal active ingredient or herbicide that can be contained or mixed in the herbicide of the present invention is not particularly limited, and examples thereof include the following.

(a) aryloxyphenoxypropionic acid ester-based ingredients such as clodinafop-propargyl, cyhalofop-butyl, diclofop-methyl, fenoxaprop-P-ethyl, fluazifop-P, fluazifop-P-butyl, haloxyfop-methyl, pyriphenop-sodium, propaquizafop, quizalofop-P-ethyl and metamifop; cyclohexanedione-based ingredients such as alloxydim, butroxydim, clethodim, cycloxydim, profoxydim, sethoxydim, tepraloxydim and tralkoxydim; phenylpyrazolin-based ingredients such as pinoxaden; and other ingredients that are said to exhibit herbicidal effects by inhibiting acetyl CoA carboxylase of plants.

(b) sulfonylurea-based ingredients such as amidosulfuron, azimsulfuron, bensulfuron-methyl, chlorimuron-ethyl, chlorsulfuron, cinosulfuron, cyclosulfamuron, ethametsulfuron-methyl, ethoxysulfuron, flazasulfuron, flupyrsulfuron, foramsulfuron, halosulfuron-methyl, imazosulfuron, iodosulfuron-methyl, mesosulfuron, mesosulfuron-methyl, metsulfuron-methyl, nicosulfuron, oxasulfuron, primisulfuron, prosulfuron, pyrazosulfuron-ethyl, rimsulfuron, sulfometuron-methyl, sulfosulfuron, thifensulfuron-methyl, triasulfuron, tribenuron-methyl, trifloxysulfuron, triflusulfuron-methyl, tritosulfuron, orthosulfamuron, propyrisulfuron, flucetosulfuron, metazosulfuron, methiopyrsulfuron, monosulfuron-methyl, orthosulfuron and iofensulfuron; imidazolinone-based ingredients such as imazapic, imazamethabenz, imazamox-ammonium, imazapyr, imazaquin and imazethapyr; triazolopyrimidine sulfonamide-based ingredients such as cloransulam-methyl, diclosulam, florasulam, flumetsulam, metosulam, penoxsulam, pyroxsulam and metosulfam; pyrimidinyl(thio)benzoate-based ingredients such as bispyribac-sodium, pyribenzoxim, pyriftalid, pyrithiobac-sodium, pyriminobac-methyl and pyrimisulfan; sulfonyl amino carbonyl triazolinone-based ingredients such as flucarbazone, propoxycarbazone and thiencarbazone-methyl; sulfonanilide-based ingredients such as triafamone; and other ingredients that are said to exhibit herbicidal effects by inhibiting acetolactate synthase (ALS) (acetohydroxy acid synthase (AHAS)) of plants.

(c) triazine-based ingredients such as ametryn, atrazine, cyanazine, desmetryne, dimethametryn, prometon, prometryn, propazine-based ingredients (propazine), CAT (simazine), simetryn, terbumeton, terbuthylazine, terbutryne, trietazine, atratone and cybutryne; triazinone-based ingredients such as hexazinone, metamitron and metribuzin; triazolinone-based ingredients such as amicarbazone; uracil-based ingredients such as bromacil, lenacil and terbacil; pyridazinone-based ingredients such as PAC (chloridazon); carbamate-based ingredients such as desmedipham, phenmedipham and swep; urea-based ingredients such as chlorobromuron, chlorotoluron, chloroxuron, dimefuron, DCMU (diuron), ethidimuron, fenuron, fluometuron, isoproturon, isouron, linuron, methabenzthiazuron, metobromuron, metoxuron, monolinuron, neburon, siduron, tebuthiuron, metobenzuron and karbutilate; amide-based ingredients such as DCPA (propanil) and CMMP (pentanochlor); anilide-based ingredients such as cypromid; nitrile-based ingredients such as bromofenoxim, bromoxynil and ioxynil; benzothiadiazinone-based ingredients such as bentazon; phenylpyridazine-based ingredients such as pyridate and pyridafol; and other ingredients that are said to exhibit herbicidal effects by inhibiting photosynthesis of plants such as methazole.

(d) bipyridylium-based ingredients such as diquat and paraquat; and other ingredients that are said to become free radicals themselves in plants and generate active oxygen to exhibit fast-acting herbicidal effects.

(e) diphenyl ether-based ingredients such as acifluorfen-sodium, bifenox, chlomethoxynil (chlomethoxyfen), fluoroglycofen, fomesafen, halosafen, lactofen, oxyfluorfen, nitrofen and ethoxyfen-ethyl; phenylpyrazole-based ingredients such as fluazolate and pyraflufen-ethyl; N-phenylphthalimide-based ingredients such as cinidon-ethyl, flumioxazin, flumiclorac-pentyl and chlorphthalim; thiadiazole-based ingredients such as fluthiacet-methyl and thidiazimin; oxadiazole-based ingredients such as oxadiazon and oxadiargyl; triazolinone-based ingredients such as azafenidin, carfentrazone-ethyl, sulfentrazone and bencarbazone; oxazolidinedione-based ingredients such as pentoxazone; pyrimidinedione-based ingredients such as benzfendizone and butafenacil; sulfonylamide-based ingredients such as saflufenacil; pyridazine-based ingredients such as flufenpyr-ethyl; and other ingredients that are said to exhibit herbicidal effects by inhibiting chlorophyll biosynthesis in plants and abnormally accumulating photosensitizing peroxide substances in plant bodies, such as pyrachlonil, profluazol, tiafenacil and trifludimoxazin.

(f) pyridazinone-based ingredients such as norflurazon and metflurazon; pyridinecarboxamide-based ingredients such as diflufenican and picolinafen; triketone-based ingredients such as mesotrione, sulcotrione, tefuryltrione, tembotrione, bicyclopyrone and fenquinotrione; isoxazole-based ingredients such as isoxachlortole and isoxaflutole; pyrazole-based ingredients such as benzofenap, pyrazolate (pyrazolynate), pyrazoxyfen, topramezone, pyrasulfotole and tolpyralate; triazole-based ingredients such as ATA (amitrol); isoxazolidinone-based ingredients such as clomazone; diphenyl ether-based ingredients such as aclonifen; and other ingredients that are said to exhibit herbicidal effects by inhibiting the biosynthesis of plant pigments such as carotenoids characterized by a bleaching action such as beflubutamid, fluridone, flurochloridone, flurtamone, benzobicyclone, methoxyphenone and ketospiradox.

(g) glycine-based ingredients such as glyphosate, glyphosate-ammonium, glyphosate-isopropylamine and glyphosate trimesium (sulfosate); and other ingredients inhibiting EPSP synthase

(h) phosphinic acid-based ingredients inhibiting glutamine synthetase such as glufosinate, glufosinate-ammonium and bialaphos (bilanafos), and

other ingredients that are said to exhibit herbicidal effects by inhibiting the amino acid biosynthesis of plants.

(i) carbamate-based ingredients such as asulam; and other ingredients inhibiting DHP (dihydropteroate) synthase

(j) dinitroaniline-based ingredients such as bethrodine (benfluralin), butralin, dinitramine, ethalfluralin, oryzalin, pendimethalin, trifluralin, nitralin and prodiamine; phosphoroamidate-based ingredients such as amiprofos-methyl and butamifos; pyridine-based ingredients such as dithiopyr and thiazopyr; benzamide-based ingredients such as propyzamide and tebutam; benzoic acid-based ingredients such as chlorthal and TCTP (chlorthal-dimethyl); carbamate-based ingredients such as IPC (chlorpropham), propham, carbetamide and barban; arylalanine-based ingredients such as flamprop-M and flamprop-M-isopropyl; chloroacetamide-based ingredients such as acetochlor, alachlor, butachlor, dimethachlor, dimethenamid, dimethenamid-P, metazachlor, metolachlor, S-metolachlor, pethoxamid, pretilachlor, propachlor, propisochlor and thenylchlor; acetamide-based ingredients such as diphenamid, napropamide and naproanilide; oxyacetamide-based ingredients such as flufenacet and mefenacet; tetrazolinone-based ingredients such as fentrazamide; and other ingredients that are said to exhibit herbicidal effects by inhibiting the microtubule polymerization, microtubule formation and cell division of plants or by inhibiting the biosynthesis of very long chain fatty acids (VLCFA), such as anilofos, indanofan, cafenstrole, piperophos, methiozolin, fenoxasulfone, pyroxasulfone and ipfencarbazone.

(k) nitrile-based ingredients such as DBN (dichlobenil) and DCBN (chlorthiamid); benzamide-based ingredients such as isoxaben; triazolocarboxamide-based ingredients such as flupoxam; quinoline carboxylic acid-based ingredients such as quinclorac; and other ingredients that are said to exhibit herbicidal effects by inhibiting the cell wall (cellulose) synthesis such as triaziflam and indaziflam.

(l) dinitrophenol-based ingredients such as DNOC, DNBP (dinoseb) and dinoterb; and other ingredients that are said to exhibit herbicidal effects by uncoupling (membrane disruption).

(m) thiocarbamate-based ingredients such as butylate, hexylthiocarbam (cycloate), dimepiperate, EPTC, esprocarb, molinate, orbencarb, pebulate, prosulfocarb, benthiocarb (thiobencarb), tiocarbazil, triallate, vernolate and diallate; phosphorodithioate-based ingredients such as SAP (bensulide); benzofuran-based ingredients such as benfuresate and ethofumesate; chlorocarbonic acid-based ingredients such as TCA, DPA (dalapon) and tetrapion (flupropanate); and other ingredients that are said to exhibit herbicidal effects by inhibiting the lipid biosynthesis of plants.

(n) phenoxycarboxylic acid-based ingredients such as clomeprop, 2,4-PA (2,4-D), 2,4-DB, dichlorprop, MCPA, MCPB and MCPP (mecoprop); benzoic acid-based ingredients such as chloramben, MDBA (dicamba) and TCBA (2,3,6-TBA); pyridinecarboxylic acid-based ingredients such as clopyralid, aminopyralid, fluroxypyr, picloram, triclopyr and halauxifen; quinoline carboxylic acid-based ingredients such as quinclorac and quinmerac; phthalamate semicarbazone-based ingredients such as NPA (naptalam) and diflufenzopyr; and other ingredients that are said to exhibit herbicidal effects by disturbing the hormone action of plants such as benazolin, diflufenzopyr, fluroxypyr, chlorflurenol, aminocyclopyrachlor, and DAS534.

(o) arylaminopropionic acid-based ingredients such as flamprop-M-methyl/isopropyl (flamprop-isopropyl); pyrazolium-based ingredients such as difenzoquat; organic arsenic-based ingredients such as DSMA and MSMA; and other herbicides such as bromobutide, chlorflurenol, cinmethylin, cumyluron, dazomet, daimuron, methyl-dymron, etobenzanid, fosamine, oxaziclomefone, oleic acid, pelargonic acid, pyributicarb, endothall, chlorates (sodium chlorate), metam, quinoclamine, cyclopyrimorate, tridiphane and clacyfos.

Examples of the phytotoxicity reducing agent (safener) that can be used in the present invention include benoxacor, cloquintocet, cloquintocet-mexyl, cyometrinil, cyprosulfamide, dichlormid, dicyclonon, dietholate, fenchlorazole, fenchlorazole-ethyl, fenclorim, flurazole, fluxofenim, furilazole, isoxadifen, isoxadifen-ethyl, mefenpyr, mefenpyr-diethyl, mephenate, naphthalic anhydride and oxabetrinil.

EXAMPLES

[Formulation Examples]

Although some formulation examples relating to the herbicide of the present invention are shown, the compounds

of the present invention, additives and addition ratios are not limited only to those in the present examples and can be changed in a wide range. The term "part" in the formulation examples indicates "part by weight".

(Formulation Example 1) Wettable powder

| | |
|---|---|
| Compound of the present invention | 20 parts |
| White carbon | 20 parts |
| Diatomaceous earth | 52 parts |
| Sodium alkyl sulfate | 8 parts |

The above components are mixed uniformly and finely pulverized to obtain a wettable powder containing 20% of an active ingredient.

(Formulation Example 2) Emulsion

| | |
|---|---|
| Compound of the present invention | 20 parts |
| Xylene | 55 parts |
| Dimethylformamide | 15 parts |
| Polyoxyethylene phenyl ether | 10 parts |

The above components are mixed and dissolved to obtain an emulsion containing 20% of an active ingredient.

(Formulation Example 3) Granule

| | |
|---|---|
| Compound of the present invention | 5 parts |
| Talc | 40 parts |
| Clay | 38 parts |
| Bentonite | 10 parts |
| Sodium alkyl sulfate | 7 parts |

The above components are uniformly mixed and finely pulverized, and then granulated into a granular form having a diameter of 0.5 to 1.0 mm to obtain a granule containing 5% of an active ingredient.

Next, synthesis examples will be shown. However, the present invention is not limited to the following synthesis examples.

[Synthesis Example 1]

Synthesis of 1-methoxy-6-(2-((2-methoxyethoxy)methyl)-6-(trifluoromethyl)pyridin-3-yl)-3-methyl-7-oxa-3,4-diazabicyclo [4.1.0] hept-4-en-2-one [1-methoxy-6-(2-((2-methoxyethoxy)methyl)-6-(trifluoromethyl)pyridin-3-yl)-3-methyl-7-oxa-3,4-diazabicyclo [4.1.0] hept-4-en-2-one] (Compound No. A-5)

(Step 1-1)

Synthesis of t-butyl(2-((2-methoxyethoxy)methyl)-6-(trifluoromethyl)pyridin-3-yl) carbamate [tert-butyl(2-((2-methoxyethoxy)methyl)-6-(trifluoromethyl)pyridin-3-yl) carbamate]

[Chemical Formula 9]

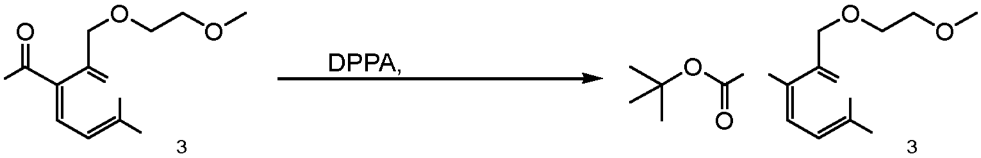


2-((2-methoxyethoxy)methyl)-6-(trifluoromethyl) nicotinic acid [2-((2-methoxyethoxy)methyl)-6-(trifluoromethyl) nicotinic acid] (2.10 g) was dissolved in tetrahydrofuran (75 mL), and the resulting mixture was stirred at room temperature. Diphenylphosphoryl azide (3.10 g) and triethylamine (0.87 g) were sequentially added thereto, and the resulting mixture was stirred at 60°C for 6 hours.

After cooling the reaction solution to room temperature, t-butyl alcohol was added thereto, and the resulting mixture was stirred at 60°C for 18 hours. Water was poured thereinto, and the resulting mixture was extracted with ethyl acetate. The obtained organic layer was washed with saturated brine, dried over anhydrous magnesium sulfate, and filtered. The filtrate was concentrated under reduced pressure, and the obtained residue was purified by silica gel column chromatography to obtain a desired product (1.52 g).

(Step 1-2)

Synthesis of 2-((2-methoxyethoxy)methyl)-6-(trifluoromethyl)pyridin-3-amine [2-((2-methoxyethoxy)methyl)-6-(trifluoromethyl)pyridin-3-amine]

[Chemical Formula 10]

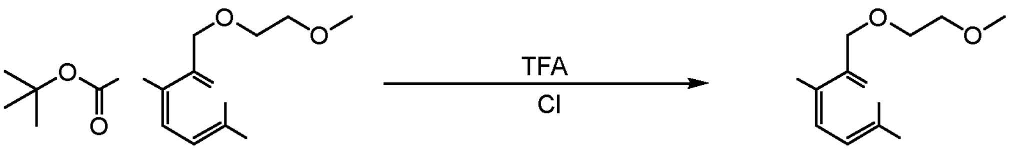


t-Butyl(2-((2-methoxyethoxy)methyl)-6-(trifluoromethyl)pyridin-3-yl) carbamate [tert-butyl(2-((2-methoxyethoxy)methyl)-6-(trifluoromethyl)pyridin-3-yl) carbamate] (1.52 g) was dissolved in methylene chloride (4.3 mL), and the resulting mixture was stirred at room temperature. Trifluoroacetic acid (4.9 g) was added thereto, and the resulting mixture was stirred at the same temperature for 3 hours.

The reaction solution was concentrated under reduced pressure, and the obtained residue was purified by silica gel column chromatography to obtain a desired product (1.13 g).

(Step 1-3)

Synthesis of 3-bromo-2-((2-methoxyethoxy)methyl)-6-(trifluoromethyl) pyridine [3-bromo-2-((2-methoxyethoxy)methyl)-6-(trifluoromethyl) pyridine]

[Chemical Formula 11]

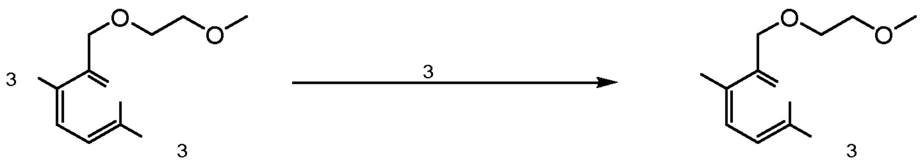

2-((2-methoxyethoxy)methyl)-6-(trifluoromethyl)pyridin-3-amine [2-((2-methoxyethoxy)methyl)-6-(trifluoromethyl)pyridin-3-amine] (1.13 g) was dissolved in acetonitrile (45 mL), and after adding copper(II) bromide (4.9 g) thereto, the resulting mixture was stirred at 65°C. t-Butyl nitrite (0.93 g) was slowly added dropwise thereto, and the resulting mixture was further stirred at the same temperature for 2 hours.

Hydrochloric acid was poured into the reaction solution, and the resulting mixture was extracted with ethyl acetate. The obtained organic layer was washed with saturated brine, dried over anhydrous magnesium sulfate, and filtered. The filtrate was concentrated under reduced pressure, and the obtained residue was purified by silica gel column chromatography to obtain a desired product (0.78 g).

(Step 1-4)

Synthesis of 4-methoxy-5-(2-((2-methoxyethoxy)methyl)-6-(trifluoromethyl)pyridin-3-yl)-2-methylpyridazin-3(2H)-one [4-methoxy-5-(2-((2-methoxyethoxy) methyl)-6-(trifluoromethyl)pyridin-3-yl)-2-methylpyridazin-3(2H)-one]

[Chemical Formula 12]

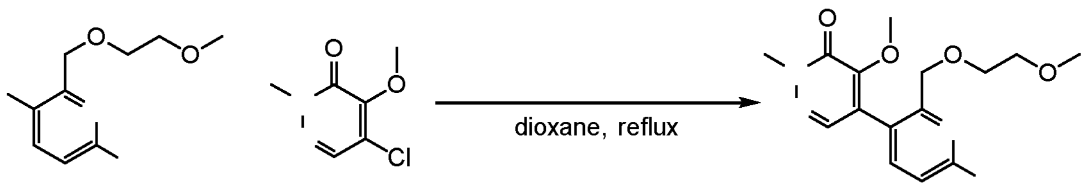


3-bromo-2-((2-methoxyethoxy)methyl)-6-(trifluoromethyl) pyridine [3-bromo-2-((2-methoxyethoxy)methyl)-6-(trifluoromethyl) pyridine] (1.13 g) was dissolved in dioxane (25 mL), and the resulting mixture was stirred at room temperature. 4-methoxy-2-methyl-5-(4,4,5,5-tetramethyl-1,3,2-dioxaborolan-2-yl) pyridazin-3(2H)-one [4-methoxy-2-methyl-5-(4,4,5,5-tetramethyl-1,3,2-dioxaborolan-2-yl) pyridazin-3(2H)-one] (0.99 g), cesium fluoride (0.68 g) and a [1,1'-bis (diphenylphosphino) ferrocene] palladium (II) dichloride-dichloromethane adduct (0.20 g) were sequentially added thereto, and the resulting mixture was heated under reflux overnight.

After filtering the reaction solution, the filtrate was concentrated under reduced pressure, and the obtained residue was purified by silica gel column chromatography to obtain a desired product (0.93 g).

(Step 1-5)

Synthesis of 4-hydroxy-5-(2-((2-methoxyethoxy)methyl)-6-(trifluoromethyl)pyridin-3-yl)-2-methylpyridazin-3(2H)-one [4-hydroxy-5-(2-((2-methoxyethoxy)methyl)-6-(trifluoromethyl)pyridin-3-yl)-2-methylpyridazin-3 (2H)-one]

[Chemical Formula 13]

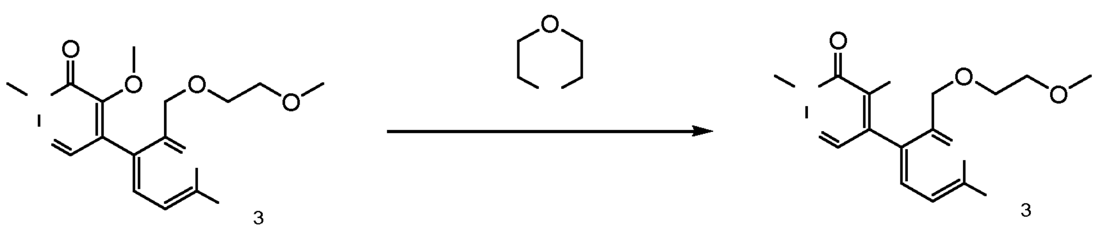

4-Methoxy-5-(2-((2-methoxyethoxy)methyl)-6-(trifluoromethyl)pyridin-3-yl)-2-methylpyridazin-3(2H)-one [4-methoxy-5-(2-((2-methoxyethoxy)methyl)-6-(trifluoromethyl)pyridin-3-yl)-2-methylpyridazin-3(2H)-one] (0.93 g) was dissolved in morpholine (5 mL), and the resulting mixture was stirred at 110°C for 1 hour.

The reaction solution was concentrated under reduced pressure, and hydrochloric acid was added thereto. This suspension was filtered to obtain a desired product (0.31 g).

(Step 1-6)

Synthesis of 1-methoxy-6-(2-((2-methoxyethoxy)methyl)-6-(trifluoromethyl)pyridin-3-yl)-3-methyl-7-oxa-3,4-diazabicyclo [4.1.0] hept-4-en-2-one [1-methoxy-6-(2-((2-methoxyethoxy)methyl)-6-(trifluoromethyl)pyridin-3-yl)-3-methyl-7-oxa-3,4-diazabicyclo [4.1.0] hept-4-en-2-one]

[Chemical Formula 14]

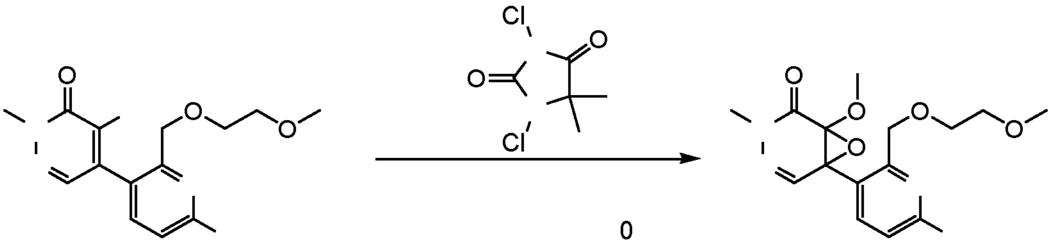


4-Hydroxy-5-(2-((2-methoxyethoxy)methyl)-6-(trifluoromethyl)pyridin-3-yl)-2-methylpyridazine-3(2H)-one [4-hydroxy-5-(2-((2-methoxyethoxy)methyl)-6-(trifluoromethyl)pyridin-3-yl)-2-methylpyridazin-3(2H)-one] (0.26 g) was dissolved in N,N-dimethylformamide (1.5 mL), and the resulting mixture was stirred at room temperature. 1,3-dichloro-5,5-dimethylhydantoin (0.17 g) was added thereto, and the resulting mixture was stirred at the same temperature for 2 hours.

After cooling the reaction solution to 0°C, methanol (3.6 mL) and sodium methoxide (0.12 g) were sequentially added, and the resulting mixture was stirred at the same temperature for 1 hour. The above reaction solution was concentrated under reduced pressure, and after pouring water thereinto, the resulting mixture was extracted with ethyl acetate. The obtained organic layer was washed with saturated brine, dried over anhydrous magnesium sulfate, and filtered. The filtrate was concentrated under reduced pressure, and the obtained residue was purified by silica gel column chromatography to obtain a desired product (0.20 g).

Table 1 shows an example of the compound of the present invention produced by the same method as in the above synthesis example. At the same time, the physical properties of the compound are also shown. Me represents a methyl group and tBu represents a t-butyl group.

[Table 1]

Table 1

| Compound No. | Structure | Physical properties |
|---|---|---|
| A-1 | 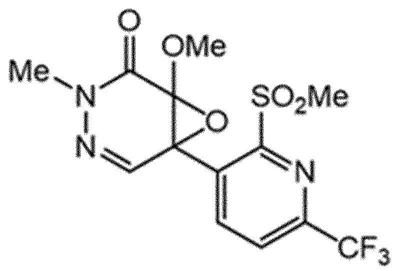 | * |

(continued)

| Compound No. | Structure | Physical properties |
|---|---|---|
| A-2 | 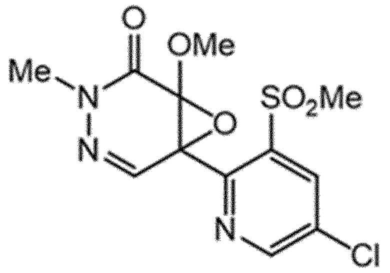 | * |
| A-3 | 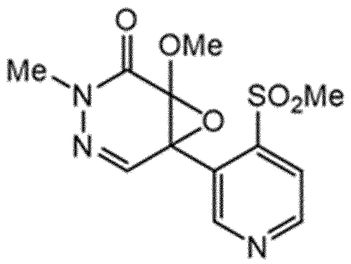 | * |
| A-4 | 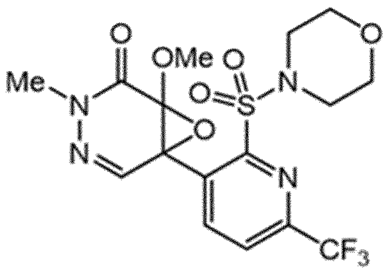 | * |
| A-5 | 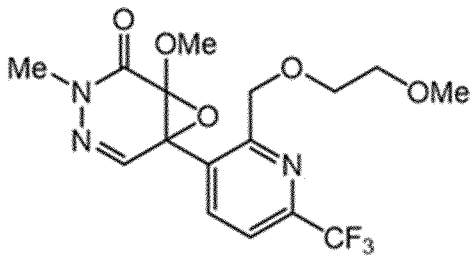 | * |

[Table 2]

| Table 1 (continued) | | |
|---|---|---|
| Compound No. | Structure | Physical properties |
| A-6 | 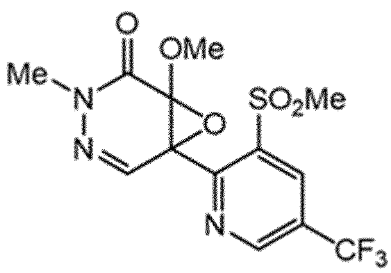 | * |

(continued)

| Table 1 (continued) | | |
|---|---|---|
| Compound No. | Structure | Physical properties |
| A-7 | 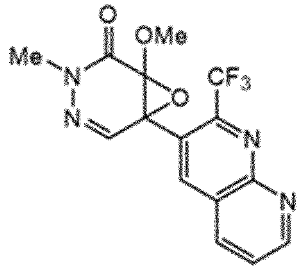 | m.p. 203-210°C |
| A-8 | 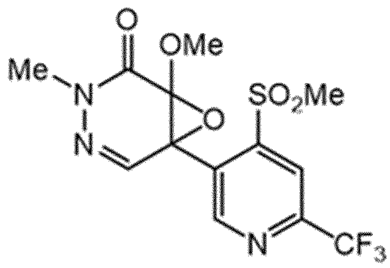 | * |
| A-9 | 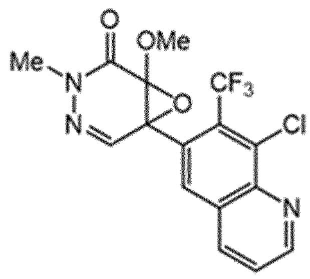 | m.p. 238-239°C |
| A-10 | 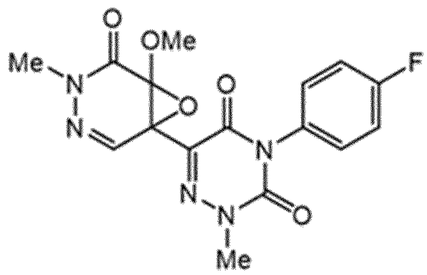 | m.p. 180-182°C |

[Table 3]

| Compound No. | Structure | Physical properties |
|---|---|---|
| A-11 | 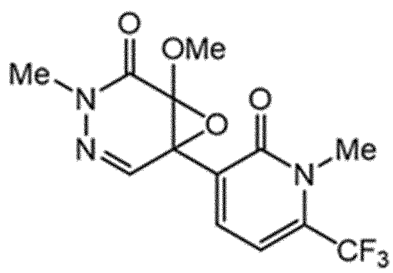 | m.p. 128-130°C |

(continued)

| Compound No. | Structure | Physical properties |
|---|---|---|
| A-12 | 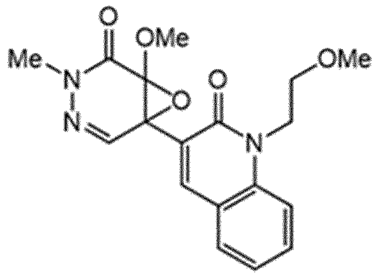 | * |
| A-13 | 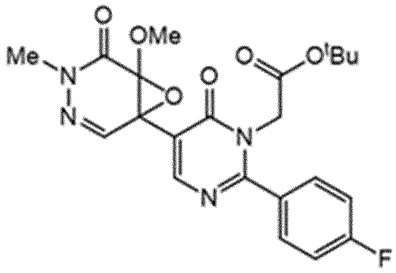 | * |
| A-14 | 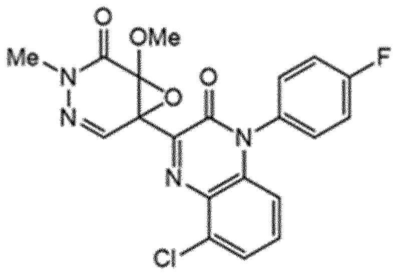 | * |
| A-15 | 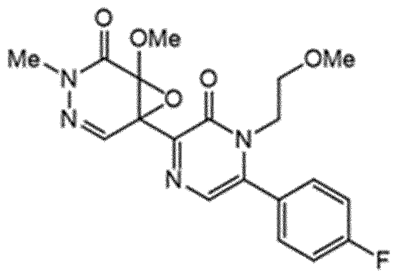 | * |

[Table 4]

| Compound No. | Structure | Physical properties |
|---|---|---|
| A-16 | 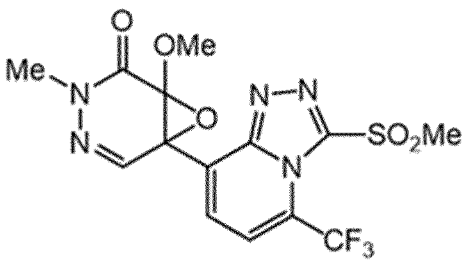 | * |

(continued)

| Compound No. | Structure | Physical properties |
|---|---|---|
| A-17 | 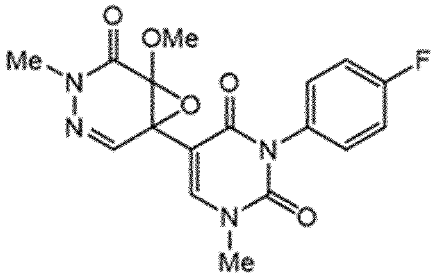 | m.p. 191-194°C |
| A-18 | 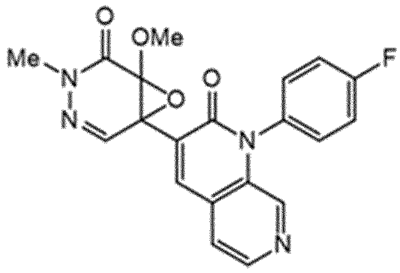 | m.p. 175-179°C |
| A-19 | 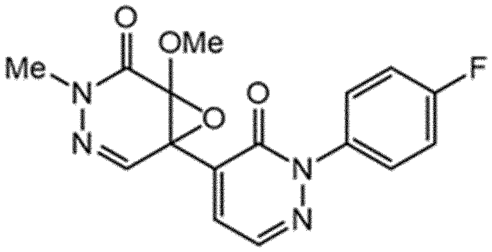 | * |
| A-20 | 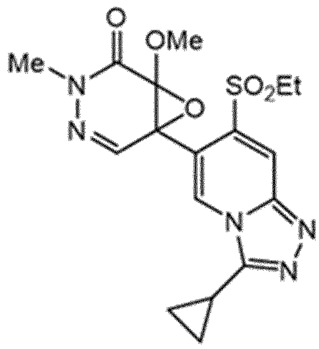 | * |

[Table 5]

| Table 1 (continued) | | |
|---|---|---|
| Compound No. | Structure | Physical properties |
| A-21 | 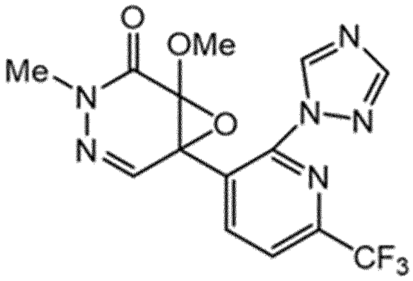 | * |
| A-22 | 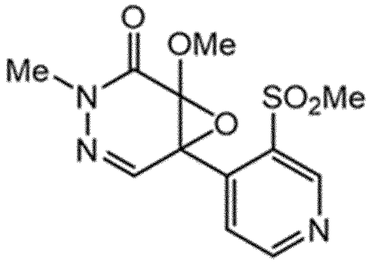 | * |
| A-23 | 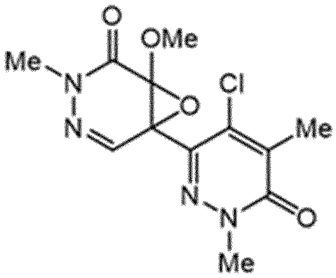 | m.p. 153-155°C |
| A-24 | 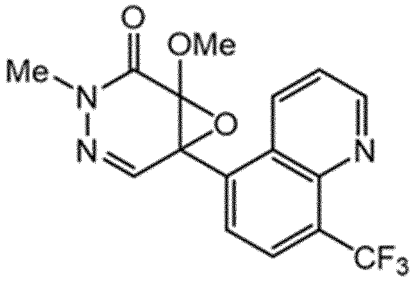 | m.p. 171-172°C |
| A-25 | 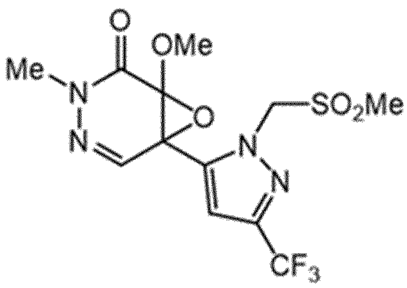 | * |

[Table 6]

| Table 1 (continued) | | |
|---|---|---|
| Compound No. | Structure | Physical properties |
| A-26 | 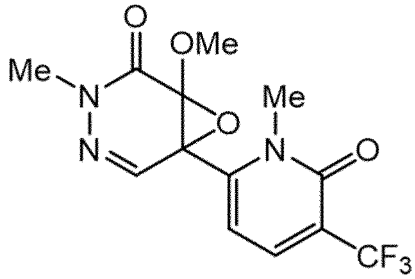 | |

Among the compounds described in Table 1, the compounds marked with asterisk (*) in the column of physical properties were compounds having properties of amorphous or viscous oil. The $^1$H-NMR data thereof are shown below.

Compound A-1: $^1$H-NMR (400 MHz, $CDCl_3$): δ3.30 (s, 3H), 3.49 (s, 3H), 3.71 (s, 3H), 7.33 (s, 1H), 7.99 (d, 1H), 8.37 (d, 1H).

Compound A-2: $^1$H-NMR (400 MHz, $CDCl_3$): δ3.18 (s, 3H), 3.50 (s, 3H), 3.80 (s, 3H), 7.23 (s, 1H), 8.30 (s, 1H), 8.91 (s, 1H).

Compound A-3: $^1$H-NMR (400 MHz, $CDCl_3$): δ3.10 (s, 3H), 3.48 (s, 3H), 3.75 (s, 3H), 7.20 (s, 1H), 7.82 (d, 1H), 8.97 (d, 1H), 9.09 (s, 1H).

Compound A-4: $^1$H-NMR (400 MHz, $CDCl_3$): δ3.35-3.50 (m, 2H), 3.53 (s, 3H), 3.55-3.61 (m, 2H), 3.68-3.81 (m, 7H), 7.35 (s, 1H), 7.94 (d, J = 8.4 Hz, 1H), 8.33 (d, J = 8.4 Hz, 1H)

Compound A-5: $^1$H-NMR (400 MHz, $CDCl_3$): δ3.32 (s, 3H), 3.44-3.46 (m, 2H), 3.52 (s, 3H), 3.59-3.65 (m, 2H), 3.71 (s, 3H), 4.74-4.89 (m, 2H), 7.34 (s, 1H), 7.70 (d, 1H), 8.03 (d, 1H).

Compound A-6: $^1$H-NMR (400 MHz, $CDCl_3$): δ3.24 (s, 1.5H), 3.45 (s, 1.5H), 3.53 (s, 1.5H), 3.54 (s, 1.5H), 3.77 (s, 1.5H), 3.84 (s, 1.5H), 7.25 (s, 0.5H), 7.38 (s, 0.5H), 8.56 (d, 0.5H), 8.71 (d, 0.5H), 9.09 (d, 0.5H), 9.23 (d, 0.5H).

Compound A-8: $^1$H-NMR (400 MHz, $CDCl_3$): δ3.18 (s, 3H), 3.53 (s, 3H), 3.81 (s, 3H), 7.22 (s, 1H), 8.21 (s, 1H), 9.22 (s, 1H).

Compound A-12: $^1$H-NMR (400 MHz, $CDCl_3$): δ3.35 (s, 3H), 3.51 (s, 3H), 3.66 (s, 3H), 3.75 (t, 2H), 4.53 (t, 2H), 7.34 (s, 1H), 7.26-7.33 (m, 1H), 7.42 (s, 1H), 7.55-7.68 (m, 3H), 7.93 (s, 1H).

Compound A-13: $^1$H-NMR (400 MHz, $CDCl_3$): δ1.44 (s, 9H), 3.50 (s, 3H), 3.75 (s, 3H), 4.47-4.59 (m, 2H), 7.18-7.22 (m, 2H), 7.44 (s, 1H), 7.54-7.58 (m, 2H), 8.14 (s, 1H).

Compound A-14: $^1$H-NMR (400 MHz, $CDCl_3$): δ3.33 (s, 3H), 3.38 (s, 3H), 6.67-6.69 (m, 1H), 7.29-7.50 (m, 6H), 7.72 (s, 1H).

Compound A-15: $^1$H-NMR (400 MHz, $CDCl_3$): δ3.18 (s, 3H), 3.51 (s, 3H), 3.59-3.64 (m, 2H), 3.81 (s, 3H), 4.08-4.11 (m, 2H), 7.19-7.23 (m, 2H), 7.36 (s, 1H), 7.44-7.49 (m, 1H + 2H).

Compound A-16: $^1$H-NMR (400 MHz, $CDCl_3$): δ3.57 (s, 3H), 3.74 (s, 3H), 3.76 (m, 2H), 7.57 (s, 1H), 7.71 (m, 2H).

Compound A-19: $^1$H-NMR (400 MHz, $CDCl_3$): δ3.48 (s, 3H), 3.75 (s, 3H), 7.16 (t, 2H), 7.41 (d, 1H), 7.44 (s, 1H), 7.64 (dd, 2H), 8.03 (d, 1H).

Compound A-20: $^1$H-NMR (400 MHz, $CDCl_3$): δ1.34 (t, 3H), 3.03 (q, 2H), 3.78 (s, 3H), 4.26 (s, 3H), 7.81 (s, 1H), 7.97 (s, 1H).

Compound A-21: $^1$H-NMR (400 MHz, $CDCl_3$): δ3.57 (s, 3H), 3.70 (s, 3H), 7.15 (s, 1H), 7.82 (d, 1H), 7.99 (s, 1H), 8.36 (d, 1H), 9.24 (s, 1H).

Compound A-22: $^1$H-NMR (400 MHz, $CDCl_3$): δ3.22 (s, 3H), 3.30 (s, 3H), 3.34 (s, 3H), 7.72 (s, 1H), 8.23 (d, 1H), 8.98 (d, 1H), 9.35 (s, 1H).

Compound A-25: $^1$H-NMR (400 MHz, $CDCl_3$): δ3.02 (s, 3H), 3.50 (s, 3H), 3.80 (s, 3H), 5.46 (d, 1H), 5.64 (d, 1H), 6.78 (s, 1H), 7.44 (s, 1H).

(Evaluation of herbicidal effects)

Next, the following test examples show that the compound of the present invention is useful as an active ingredient of a herbicide.

(Test Example 1)

(1) Preparation of test emulsion

POA allylphenyl ether (4.1 parts by weight), POE-POP glycol (1 part by weight), POE sorbitan laurate (0.8 parts by weight), glycerin (2.6 parts by weight), dimethylformamide (65.9 parts by weight), N-methylpyrrolidone (5.1 parts by weight), cyclohexanone (15.4 parts by weight), and aromatic hydrocarbons (5.1 parts by weight) were mixed and dissolved to prepare an emulsion. The compound of the present invention (4 mg) was dissolved in this emulsion (100 μL) to prepare a test emulsion. POA means "polyoxyalkylene", POE means "polyoxyethylene", and POP means "polyoxypropylene".

(2) Soil treatment

A 70 $cm^2$ pot was filled with soil, and seeds of Digitaria ciliaris, Setaria faberi, Abutilon theophrasti and Amaranthus blitum were sown into the surface layer which was covered lightly with soil. The next day, the above test emulsion was diluted so as to achieve a predetermined amount of active ingredient and sprayed on the soil surface with a small sprayer at a spray water volume of 2,860 L per hectare.

(3) Evaluation

After 4 weeks, the above ground weights of weeds in the untreated and treated areas were measured for each weed, and the weed killing rate was calculated by the following calculation formula.

(4) Calculation formula for weed killing rate

$$\text{Weed killing rate (\%)} = [(\text{above ground weight of weeds in untreated area}) - (\text{above ground weight of weeds in treated area}) / (\text{above ground weight of weeds in untreated area})] \times 100$$

(a) Digitaria ciliaris

The compounds of compound numbers A-1, A-2, A-4, A-5, A-6 and A-7 were sprayed so that the spray volume was 250 g per hectare. As a result, all the compounds showed a herbicidal activity of 80% or more with respect to Digitaria ciliaris.

(b) Setaria faberi

The compounds of compound numbers A-1, A-4 and A-25 were sprayed so that the spray volume was 250 g per hectare. As a result, all the compounds showed a herbicidal activity of 80% or more with respect to Setaria faberi.

(c) Abutilon theophrasti

The compounds of compound numbers A-1, A-2, A-4, A-5, A-6, A-7, A-8, A-23, A-24 and A-25 were sprayed so that the spray volume was 250 g per hectare. As a result, all the compounds showed a herbicidal activity of 80% or more with respect to Abutilon theophrasti.

(d) Amaranthus blitum

The compounds of compound numbers A-1, A-4, A-5, A-6, A-7, A-8, A-24 and A-25 were sprayed so that the spray volume was 250 g per hectare. As a result, all the compounds showed a herbicidal activity of 80% or more with respect to Amaranthus blitum.

Since all of those randomly selected from among the compounds of the present invention exert the above-mentioned effects, it can be understood that the compounds of the present invention including the compounds that are not exemplified are compounds having high herbicidal effects.

INDUSTRIAL APPLICABILITY

It is possible to provide a 7-oxa-3,4-diazabicyclo[4.1.0]hept-4-en-2-one compound useful as an active ingredient of a herbicide, which has a reliable weed control effect even at a low dose, causes less phytotoxicity to crops, and is highly safe for the environment; and a herbicide.

## Claims

**1.** A compound represented by a formula (I) or a salt thereof:

[Chemical Formula 1]

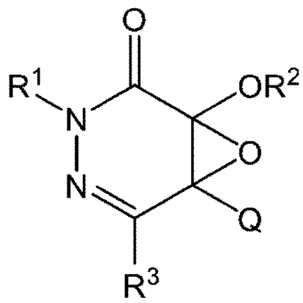

(I)

wherein

$R^1$ represents a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{3-6}$ cycloalkyl group, or a 5- to 6-membered cyclic ether group,
$R^2$ represents a substituted or unsubstituted $C_{1-6}$ alkyl group, a substituted or unsubstituted $C_{2-6}$ alkenyl group, or a substituted or unsubstituted $C_{2-6}$ alkynyl group,
$R^3$ represents a hydrogen atom, a substituted or unsubstituted $C_{1-6}$ alkyl group, a substituted or unsubstituted $C_{2-6}$ alkenyl group, a substituted or unsubstituted $C_{2-6}$ alkynyl group, a substituted or unsubstituted $C_{1-6}$ alkoxy group, a substituted or unsubstituted $C_{3-6}$ cycloalkyl group, or a substituted or unsubstituted phenyl group, and
Q represents a substituted or unsubstituted 5- to 10-membered heterocyclyl group.

**2.** A herbicide comprising at least one selected from the group consisting of the compound according to Claim 1 and a salt thereof as an active ingredient.

**3.** A method for controlling a weed, the method comprising a step of applying the compound according to Claim 1 or a salt thereof, or the herbicide according to Claim 2 to a useful plant, a weed in said useful plant, and/or a place where the useful plant grows or is growing.

## INTERNATIONAL SEARCH REPORT

International application No.
**PCT/JP2022/010651**

**A. CLASSIFICATION OF SUBJECT MATTER**

***C07D 491/044***(2006.01)i; ***A01P 13/00***(2006.01)i; ***C07D 519/00***(2006.01)i; ***A01N 43/58***(2006.01)i; ***A01N 43/653***(2006.01)i; ***A01N 43/707***(2006.01)i

FI: C07D491/044 CSP; A01N43/58 B; A01N43/707; A01N43/653 N; A01P13/00; C07D519/00 301

According to International Patent Classification (IPC) or to both national classification and IPC

**B. FIELDS SEARCHED**

Minimum documentation searched (classification system followed by classification symbols)

C07D491/044; A01P13/00; C07D519/00; A01N43/58; A01N43/653; A01N43/707

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | JP 2017-518984 A (BAYER CROPSCIENCE AG) 13 July 2017 (2017-07-13) claims, examples | 1-3 |
| A | JP 2014-528960 A (SYNGENTA LTD.) 30 October 2014 (2014-10-30) claims, examples | 1-3 |
| P, X | WO 2021/060240 A1 (NIPPON SODA CO., LTD.) 01 April 2021 (2021-04-01) claims, examples | 1-3 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 May 2022** | **24 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/010651**

| Patent document cited in search report | Publication date (day/month/year) | Patent family member(s) | Publication date (day/month/year) |
|---|---|---|---|
| JP 2017-518984 A | 13 July 2017 | US 2017/0096402 A1<br>claims, examples<br>WO 2015/177109 A1<br>EP 3145917 A1<br>CN 106536487 A | |
| JP 2014-528960 A | 30 October 2014 | US 2014/0256546 A1<br>claims, examples<br>WO 2013/050421 A1<br>EP 2763968 A1<br>CN 103874688 A | |
| WO 2021/060240 A1 | 01 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021046669 A
- WO 2013050421 A1